(19) **Europäisches Patentamt / European Patent Office / Office européen des brevets**

(11) **EP 4 338 664 A1**

(12) # EUROPEAN PATENT APPLICATION
published in accordance with Art. 153(4) EPC

(43) Date of publication:
**20.03.2024 Bulletin 2024/12**

(21) Application number: **22836772.8**

(22) Date of filing: **28.06.2022**

(51) International Patent Classification (IPC):
***A61B 5/0537*** (2021.01)

(52) Cooperative Patent Classification (CPC):
**A61B 5/00; A61B 5/0537**

(86) International application number:
**PCT/CN2022/101925**

(87) International publication number:
**WO 2023/280018 (12.01.2023 Gazette 2023/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.07.2021 CN 202110769492**

(71) Applicant: **Huawei Technologies Co., Ltd.**
**Longgang**
**Shenzhen, Guangdong 518129 (CN)**

(72) Inventors:
• **YAN, Jiabing**
  **Shenzhen, Guangdong 518129 (CN)**
• **ZHAO, Shuai**
  **Shenzhen, Guangdong 518129 (CN)**
• **YANG, Bin**
  **Shenzhen, Guangdong 518129 (CN)**
• **REN, Huichao**
  **Shenzhen, Guangdong 518129 (CN)**

(74) Representative: **Isarpatent**
**Patent- und Rechtsanwälte**
**Barth Hassa Peckmann & Partner mbB**
**Friedrichstraße 31**
**80801 München (DE)**

(54) **WEARABLE DEVICE AND PHYSIOLOGICAL PARAMETER MEASUREMENT METHOD**

(57)    A wearable device includes a device body, a flexible fixing strip connected to the device body, and a plurality of groups of electrodes. Each group of electrodes includes at least one electrode, and at least one group of the plurality of groups of electrodes is disposed on the flexible fixing strip. The plurality of groups of electrodes may measure a liver impedance of a user based on an electrical signal generated by the wearable device, so that a risk level of a user's liver can be tested based on the liver impedance of the user. In this way, the user's liver can be accurately and conveniently tested by using the wearable device, thereby improving user experience.

FIG. 5

EP 4 338 664 A1

**Description**

This application claims priority to Chinese Patent Application No. 202110769492.0, filed with the China National Intellectual Property Administration on July 7, 2021 and entitled "WEARABLE DEVICE AND PHYSIOLOGICAL PARAMETER MEASUREMENT METHOD", which is incorporated herein by reference in its entirety.

**TECHNICAL FIELD**

This application relates to the field of terminal technologies, and in particular, to a wearable device and a physiological parameter measurement method.

**BACKGROUND**

With improvement of living standards, fatty liver (fatty liver) has become the first major liver disease in humans, and a prevalence rate is gradually increasing. Formation of the fatty liver is mainly caused by pathological changes in the liver due to excessive fat accumulation in hepatocytes. In general, when steatosis occurs in 5% or more of liver tissue, the fatty liver can be diagnosed. Therefore, liver fat is an important characteristic of fatty liver detection. However, currently, a process of liver testing is too complex, and it is inconvenient to carry a test device. This is very unfriendly to a user. Therefore, at present, how to provide a device that can accurately and conveniently test a risk level of a user's liver is a technical problem that needs to be resolved urgently.

**SUMMARY**

This application provides a wearable device and a physiological parameter measurement method, to accurately and conveniently test a risk level of a user's liver.

According to a first aspect, this application provides a wearable device, including a device body, a flexible fixing strip connected to two ends of the device body, and at least two groups of electrodes, where each group of electrodes includes at least one electrode, at least one group of the at least two groups of electrodes is disposed on the flexible fixing strip, and the at least two groups of electrodes are configured to measure a physiological parameter of a user based on an electrical signal generated by the wearable device. In this way, the physiological parameter such as a liver impedance of the user can be accurately and conveniently measured by using the wearable device, to determine a risk level of a user's liver based on the measured liver impedance.

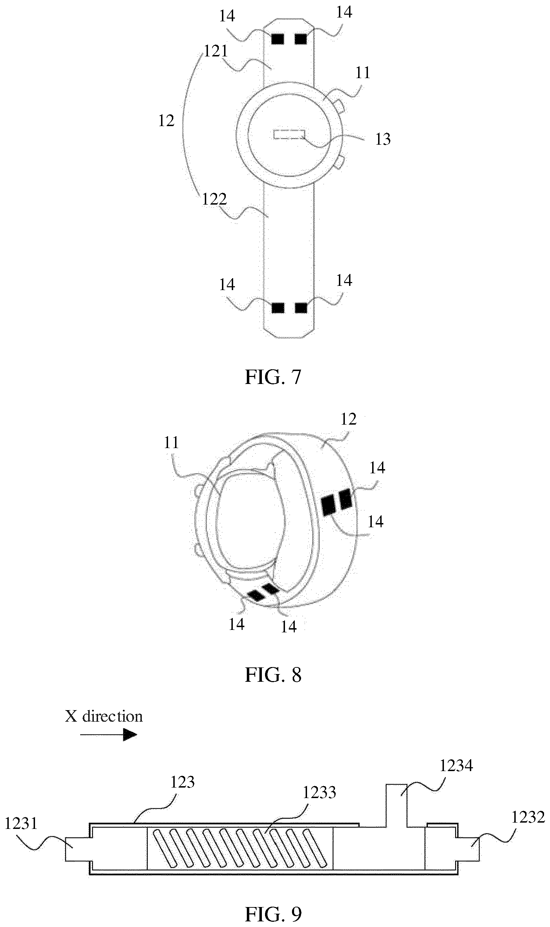

In a possible implementation, the at least two groups of electrodes include a first group of electrodes and a second group of electrodes. The flexible fixing strip includes a first flexible strip and a second flexible strip. The first flexible strip is connected to one end of the device body and the second flexible strip is connected to the other end of the device body. The first group of electrodes is disposed on the first flexible strip and the second group of electrodes is disposed on the second flexible strip. In this way, the two groups of electrodes are both disposed on the flexible fixing strip, so that the physiological parameter such as the liver impedance can be easily measured. For example, the first flexible strip may be a flexible strip 121 shown in FIG. 7, and the second flexible strip may be a flexible strip 122 shown in FIG. 7.

In a possible implementation, when both the first flexible strip and the second flexible strip are in an unfolded state, a distance between the first group of electrodes and the second group of electrodes is greater than a preset distance. In this way, when the physiological parameter such as the liver impedance is measured, one group of electrodes may be placed in an upper area of the liver, and the other group of electrodes may be placed in a lower area of the liver, so that the physiological parameter such as the liver impedance can be easily measured.

In a possible implementation, the device body includes a display screen. The first group of electrodes is located on a same side of the first flexible strip as the display screen, and the second group of electrodes is located on a side that is of the second flexible strip and that faces away from the display screen; or the first group of electrodes is located on a same side of the first flexible strip as the display screen, and the second group of electrodes is located on a same side of the second flexible strip as the display screen; or the first group of electrodes is located on a side that is of the first flexible strip and that faces away from the display screen, and the second group of electrodes is located on a side that is of the second flexible strip and that faces away from the display screen. For example, when the display screen is located on a front side of the wearable device, a side that faces away from the display screen may be understood as a rear side of the wearable device. For example, "facing away" may also be understood as "facing away", "facing away", or the like.

In a possible implementation, the at least two groups of electrodes include a first group of electrodes and a second group of electrodes, the first group of electrodes is disposed on the device body, and the second group of electrodes is disposed on flexible fixing strip. In this way, one group of electrodes is disposed on the device body, and the other group of electrodes is disposed on the flexible fixing strip, so that the physiological parameter such as the liver impedance can be easily measured.

In a possible implementation, when the flexible fixing strip is in an unfolded state, a distance between the first group of electrodes and the second group of electrodes is greater than a preset distance. In this way, when the physiological parameter such as the liver impedance is measured, one group of electrodes may be placed in an upper area of the liver, and the other group of electrodes may be placed in a lower area of the liver, so that the physiological parameter such as the liver impedance can be easily measured.

In a possible implementation, the device body includes a display screen. The first group of electrodes is located on a side that is of the device body and that faces away from the display screen, and the second group of electrodes is located on a same side of the flexible fixing strip as the display screen; or the first group of electrodes is located on a same side of the device body as the display screen, and the second group of electrodes is located on a same side of the flexible fixing strip as the display screen; or the first group of electrodes is located on a side that is of the device body and that faces away from the display screen, and the second group of electrodes is located on a side that is of the flexible fixing strip and that faces away from the display screen; or the first group of electrodes is located on a same side of the device body as the display screen, and the second group of electrodes is located on a side that is of the flexible fixing strip and that faces away from the display screen.

In a possible implementation, the flexible fixing strip is connected to the device body in an undetachable manner.

In a possible implementation, the flexible fixing strip is provided with a first cable channel, the device body is provided with a second cable channel, the first cable channel and the second cable channel are communicated with each other and are both configured to accommodate a first cable, and the first cable is configured to connect a processor of the wearable device to the electrode on the flexible fixing strip.

In a possible implementation, the flexible fixing strip is detachably connected to the device body.

In a possible implementation, the flexible fixing strip includes a first locking piece, an elastic component, a pushing member, and a second locking piece, where the first locking piece, the elastic component, the pushing member, and the second locking piece are sequentially arranged in a width direction of the flexible fixing strip. The device body is provided with a first slot to which the first locking piece is fitted and a second slot to which the second locking piece is fitted. When external force toward the first locking piece is applied to the pushing member, the pushing member presses the elastic component, to drive the second locking piece to be disengaged from the second slot and/or move toward the first locking piece.

In a possible implementation, the first locking piece is electrically connected to the at least one electrode on the flexible fixing strip, the first slot is electrically connected to a processor in the wearable device, and the first locking piece is electrically connected to the first slot; and/or the second locking piece is electrically connected to the at least one electrode on the flexible fixing strip, the second slot is electrically connected to a processor in the wearable device, and the second locking piece is electrically connected to the second slot.

In a possible implementation, the at least two groups of electrodes are two groups, and each group of electrodes includes one electrode, or each group of electrodes includes two electrodes.

In a possible implementation, the physiological parameter includes the liver impedance.

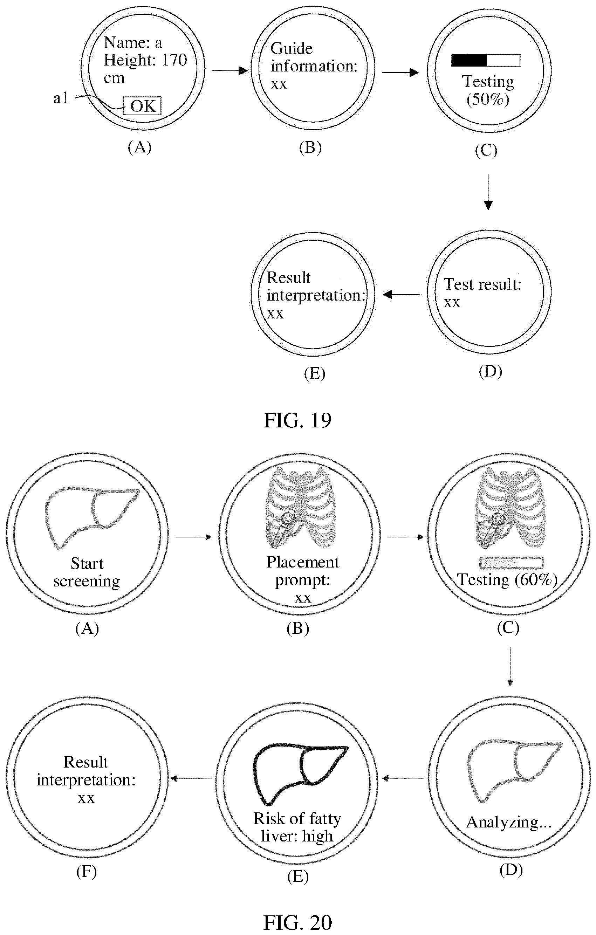

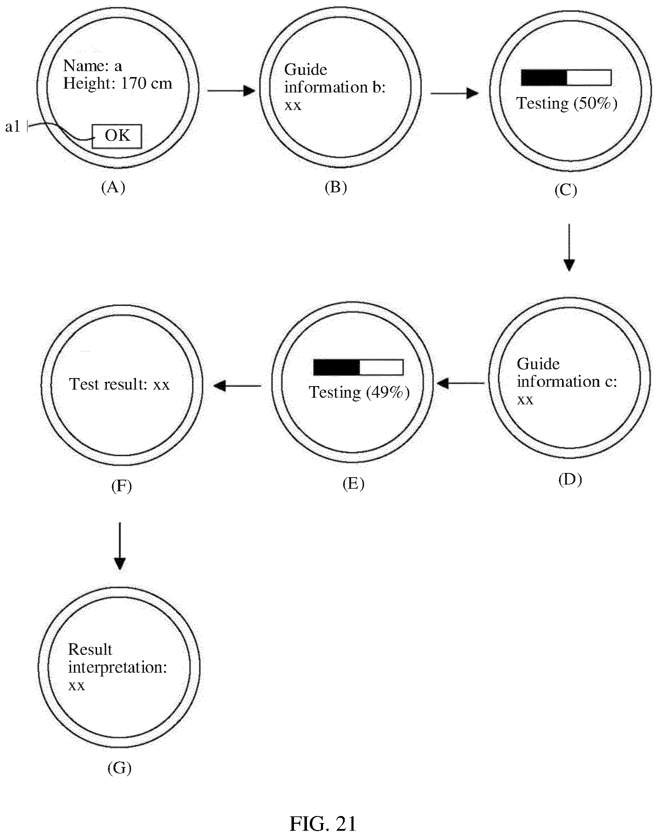

According to a second aspect, this application provides a physiological parameter measurement method, applied to a wearable device. The wearable device includes a device body, a flexible fixing strip, and at least two groups of electrodes, the flexible fixing strip is connected to two ends of the device body, the at least two groups of electrodes include a first group of electrodes and a second group of electrodes, the first group of electrodes is disposed on the flexible fixing strip, and the second group of electrodes is disposed on the flexible fixing strip or the device body. The method includes: detecting a first operation; in response to the first operation, generating a first current when the first group of electrodes is electrically connected to the second group of electrodes, where the first current has a first current value, and the first current flows through the first group of electrodes and the second group of electrodes; determining a first voltage value between the first group of electrodes and the second group of electrodes; and determining a first physiological parameter based on the first current value and the first voltage value. In this way, the physiological parameter such as a liver impedance of the user can be accurately and conveniently measured by using the wearable device, to determine a risk level of a user's liver based on the measured liver impedance. For example, the first physiological parameter may be the liver impedance. For example, the first operation may be an operation of testing the risk level of a liver. For example, the first operation may be an operation that the user may tap an "OK" button in an area a1 in (A) of FIG. 19, or an operation that the user may tap an "OK" button in an area a1 in (A) of FIG. 21.

In a possible implementation, the generating a first current; determining a first voltage value between the first group of electrodes and the second group of electrodes; and determining a first physiological parameter based on the first current value and the first voltage value further includes: generating a second current of a first frequency, where the second current has a second current value; determining a second voltage value between the first group of electrodes and the second group of electrodes; generating a third current of a second frequency, where the third current has a third current value; determining a third voltage value between the first group of electrodes and the second group of electrodes;

and determining the first physiological parameter based on the second current value, the second voltage value, the third current value, and the third voltage value. In this way, the first physiological parameter can be measured from a plurality of dimensions by using a plurality of currents with different frequencies, thereby improving accuracy of measuring the first physiological parameter.

In a possible implementation, the device body includes a display screen, and the first group of electrodes and the second group of electrodes are located on a same side of the flexible fixing strip and/or the device body as the display screen.

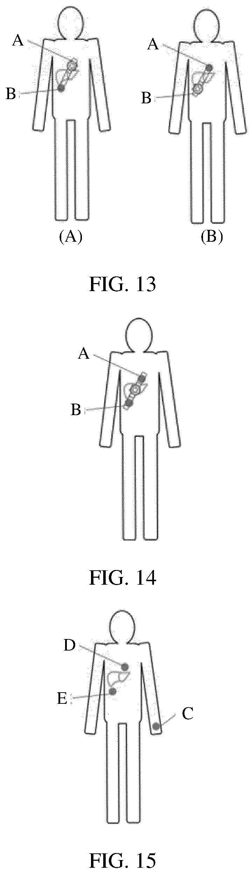

In a possible implementation, the method further includes: in response to the first operation, displaying a first interface, where the first interface indicates a user to enable the first group of electrodes and the second group of electrodes to get in contact with a first position and a second position of a user body. In this way, the user may be prompted, by using the first interface, to place an electrode at a specific position on the body, to facilitate measurement of the first physiological parameter. For example, the first operation may be an operation that the user may tap an "OK" button in an area a1 in (A) of FIG. 19. For example, the first position may be a xiphoid process position on the user body (for example, an area A in FIG. 13), and the second position may be a position below a rib on a right side of the user body (for example, an area B in FIG. 13). For example, the first physiological parameter may be the liver impedance. For example, the first interface may be an interface shown in (B) of FIG. 19.

In a possible implementation, the method further includes: determining whether the first physiological parameter falls within a preset range; and if the first physiological parameter is not within the preset range, prompting to perform re-measurement. In this way, it can be determined whether the measured first physiological parameter is consistent with the preset range, to avoid a measurement error, and prompt the user to perform re-measurement when the measurement error occurs.

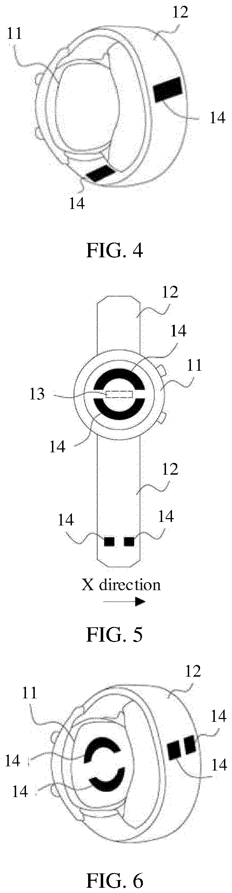

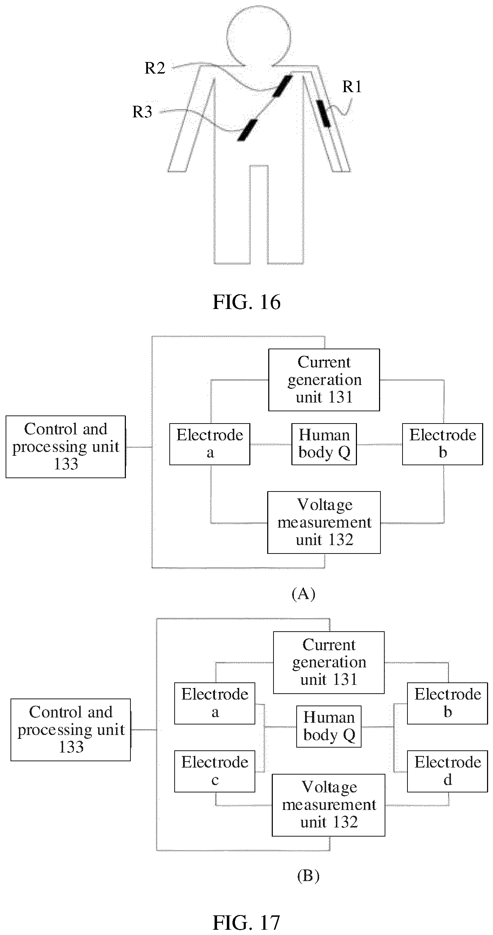

In a possible implementation, the method further includes: in response to the first operation, displaying a second interface, where the second interface indicates a user to enable the first group of electrodes to get in contact with a first position of a user body; and after the determining a first physiological parameter, the method further includes: displaying a third interface, where the third interface indicates the user to contact the first group of electrodes with a second position of the user body; generating a fourth current when the first group of electrodes is electrically connected to the second group of electrodes, where the fourth current has a fourth current value, and the fourth current flows through the first group of electrodes and the second group of electrodes; determining a fourth voltage value between the first group of electrodes and the second group of electrodes; determining a second physiological parameter based on the fourth current value and the fourth voltage value; and determining a third physiological parameter based on the first physiological parameter and the second physiological parameter, where the device body includes the display screen, the second group of electrodes is located on a side that is of the device body and that faces away from the display screen, and the first group of electrodes is located on a same side of the flexible fixing strip as the display screen. In this way, the user can measure the physiological parameter of the user when wearing the wearable device. For example, the first operation may be an operation that the user may tap an "OK" button in an area a1 in (A) of FIG. 21. For example, the second interface may be an interface shown in (B) of FIG. 21. The third interface may be an interface shown in (D) of FIG. 21. For example, the first position may be a xiphoid process position on the user body (for example, an area D in FIG. 16), and the second position may be a position below a rib on a right side of the user body (for example, an area E in FIG. 16). For example, the first physiological parameter may be R1+R2 shown in FIG. 16. The second physiological parameter may be R1+R2+R3 shown in FIG. 16, and the third physiological parameter may be R3 shown in FIG. 6.

In a possible implementation, the method further includes: determining whether the third physiological parameter falls within a preset range; and if the third physiological parameter is not within the preset range, prompting to perform re-measurement. For example, the third physiological parameter may be the liver impedance.

According to a third aspect, this application provides a physiological parameter measurement method, applied to an electronic device. The electronic device has a first group of electrodes and a second group of electrodes. The first group of electrodes is located on a first surface of the electronic device, the second group of electrodes is located on a second surface of the electronic device. The first surface and the second surface are different sides of the electronic device. The method includes: detecting a first operation; in response to the first operation, displaying a first user interface, where the first user interface indicates a user to enable a first part and a second part to get in contact with the first group of electrodes and the second group of electrodes respectively, so that the first group of electrodes is electrically connected to the second group of electrodes; when the first group of electrodes is electrically connected to the second group of electrodes, generating a first current, where the first current has a first current value; determining a first voltage value between the first group of electrodes and the second group of electrodes; determining a first physiological parameter based on the first current value and the first voltage value; determining n second physiological parameters based on the first physiological parameter, where n is a positive integer greater than or equal to 1; and obtaining a third physiological parameter based on the n second physiological parameters. In this way, the physiological parameter such as a liver impedance of the user can be accurately and conveniently measured by using the wearable device, to determine a risk level of a user's liver based on the measured liver impedance.

For example, the first part may be a left finger of the user described in FIG. 6, the second part may be a right finger of the user described in FIG. 6, the first physiological parameter may be an upper limb impedance, the n second physiological parameters may include one or more of a body fat rate, visceral fat content in a torso, or torso fat content, and the third physiological parameter may be liver fat content or a liver risk level.

In a possible implementation, the obtaining a third physiological parameter based on the n second physiological parameters specifically includes: obtaining a fourth physiological parameter; and obtaining the third physiological parameter based on the n second physiological parameters and the fourth physiological parameter. For example, the fourth physiological parameter includes a waist circumference and/or a height. In this way, the third physiological parameter is obtained with reference to the fourth physiological parameter, thereby improving accuracy of measuring the third physiological parameter.

In a possible implementation, the obtaining the third physiological parameter based on the n second physiological parameters and the fourth physiological parameter specifically includes: determining a timing variance between a currently obtained fourth physiological parameter and a previously obtained fourth physiological parameter; and obtaining the third physiological parameter based on the timing variance, the n second physiological parameters, and the currently obtained fourth physiological parameter. For example, the fourth physiological parameter is the waist circumference. In this way, the third physiological parameter is corrected based on the input timing variance of the fourth physiological parameter, thereby improving accuracy of measuring the third physiological parameter.

According to a fourth aspect, this application provides a wearable device, including at least one memory, configured to store a program; and at least one processor, configured to execute the program stored in the memory, where when the program stored in the memory is executed, the processor is configured to perform the method provided in the second aspect or the third aspect.

According to a fifth aspect, this application provides a computer storage medium, where the computer storage medium stores instructions, and when the instructions are run on a computer, the computer is enabled to perform the method provided in the second aspect or the third aspect.

According to a sixth aspect, this application provides a computer program product including instructions, where when the instructions are run on a computer, the computer is enabled to perform the method provided in the second aspect or the third aspect.

## BRIEF DESCRIPTION OF DRAWINGS

The following briefly describes the accompanying drawings that need to be used in the descriptions of embodiments or conventional technologies.

FIG. 1 is a schematic diagram of a structure of a wearable device according to an embodiment of this application;

FIG. 2 is a schematic diagram of electrode arrangement on a wearable device according to an embodiment of this application;

FIG. 3 is a schematic diagram of electrode arrangement on a flexible fixing strip of a wearable device according to an embodiment of this application;

FIG. 4 is a schematic diagram of electrode arrangement on a flexible fixing strip of a wearable device according to an embodiment of this application;

FIG. 5 is a schematic diagram of electrode arrangement on a wearable device according to an embodiment of this application;

FIG. 6 is a schematic diagram of electrode arrangement on a wearable device according to an embodiment of this application;

FIG. 7 is a schematic diagram of electrode arrangement on a wearable device according to an embodiment of this application;

FIG. 8 is a schematic diagram of electrode arrangement on a wearable device according to an embodiment of this application;

FIG. 9 is a schematic diagram of a partial structure of a flexible fixing strip on a wearable device according to an embodiment of this application;

FIG. 10 is a schematic diagram of a partial structure of a device body on a wearable device according to an embodiment of this application;

FIG. 11 is a schematic diagram of engagement between a device body and a flexible fixing strip on a wearable device according to an embodiment of this application;

FIG. 12 is a schematic diagram of a structure of a processor on a wearable device according to an embodiment of this application;

FIG. 13 is a schematic diagram of a placement position of an electrode on a wearable device according to an embodiment of this application;

FIG. 14 is a schematic diagram of a placement position of an electrode on a wearable device according to an embodiment of this application;

FIG. 15 is a schematic diagram of a placement position of an electrode on a wearable device according to an embodiment of this application;

FIG. 16 is a schematic diagram of an equivalent impedance of a human body in a process of measuring a liver impedance according to an embodiment of this application;

FIG. 17 is a schematic diagram of a principle of measuring a liver impedance according to an embodiment of this application;

FIG. 18 is a schematic diagram of a principle of measuring a liver impedance according to an embodiment of this application;

FIG. 19 is a schematic diagram of a process of testing a liver risk level according to an embodiment of this application;

FIG. 20 is a schematic diagram of an interface change of a wearable device in a process of testing a liver risk level according to an embodiment of this application;

FIG. 21 is a schematic diagram of a process of testing a liver risk level according to an embodiment of this application; and

FIG. 22 is a schematic diagram of an interface change of a wearable device in a process of testing a liver risk level according to an embodiment of this application.

## DESCRIPTION OF EMBODIMENTS

To make the objectives, technical solutions, and advantages of embodiments of this application clearer, the following describes the technical solutions of embodiments of this application with reference to the accompanying drawings.

Terms used in the following embodiments are merely intended to describe specific embodiments, but are not intended to limit this application. The terms "one", "a", "the", "the above" and "this" of singular forms used in this specification and the appended claims of this application are also intended to include expressions such as "one or more", unless otherwise specified in the context clearly. It should be further understood that in the following embodiments of this application, "at least one" and "one or more" mean one, two, or more. The term "and/or" is used for describing an association relationship between associated objects and represents that three relationships may exist. For example, A and/or B may represent the following cases: Only A exists, both A and B exist, and only B exists, where A and B may be singular or plural. The character "/" generally indicates an "or" relationship between the associated objects.

Reference to "an embodiment", "some embodiments", or the like described in this specification indicates that one or more embodiments of this application include a specific feature, structure, or characteristic described with reference to embodiments. Therefore, statements such as "in an embodiment", "in some embodiments", "in some other embodiments", and "in other embodiments" that appear at different places in this specification do not necessarily mean referring to a same embodiment. Instead, the statements mean "one or more but not all of embodiments", unless otherwise specifically emphasized in another manner. The terms "include", "have", and their variants all mean "include but are not limited to", unless otherwise specifically emphasized in another manner. The term "connection" includes a direct connection and an indirect connection, unless otherwise indicated.

The terms "first" and "second" mentioned below are merely intended for a purpose of description, and shall not be understood as an indication or implication of relative importance or implicit indication of a quantity of indicated technical features. Therefore, a feature limited by "first" or "second" may explicitly or implicitly include one or more of the features.

In embodiments of this application, the term "example", "for example", or the like is used for representing giving an example, an illustration, or a description. Any embodiment or design scheme described as "example" or "for example" in embodiments of this application should not be explained as being more preferred or having more advantages than another embodiment or design scheme. Exactly, use of the term "example", "for example", or the like is intended to present a relative concept in a specific manner.

Embodiments of this application provide a wearable device. The wearable device may measure liver fat of a user. For example, the wearable device includes but is not limited to a watch, a band, or the like.

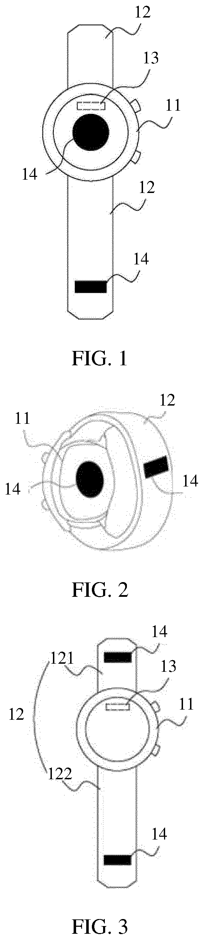

For example, FIG. 1 is a schematic diagram of a structure of a wearable device according to an embodiment of this application. As shown in FIG. 1, a wearable device 100 includes a device body 11, a flexible fixing strip 12 connected to two ends of the device body 11, a processor 13, and at least two groups of electrodes, where each group of electrodes may include at least one electrode 14. The processor 13 is disposed in the device body 11, and each electrode 14 is electrically connected to the processor 13. For example, a group of electrodes may be disposed on the device body 11, and a group of electrodes may be disposed on the flexible fixing strip 12. In addition, two groups of electrodes may be disposed on the flexible fixing strip 12, and the two groups of electrodes are arranged in spacing on the flexible fixing strip 12. In an example, a plurality of groups of electrodes on the wearable device 100 may be located on a same side of the wearable device 100, for example, all on a side on which the wearable device 100 is in contact with a user body (that is, a rear side of the wearable device 100). In addition, the plurality of groups of electrodes on the wearable device 100 may be located on different sides of the wearable device 100. For example, one group of electrodes may be located on the side on which the wearable device 100 is in contact with the user body (that is, the rear side of the wearable device 100), and another group of electrodes may be located on a side on which the wearable device 100 faces away from the user body (that is, a front side of the wearable device 100). When a group of electrodes includes a plurality of electrodes, the plurality of electrodes may be arranged in spacing in a preset distance, for example, 1 millimeter.

For example, a display screen (not shown in the figure) may be disposed on the device body 11. For ease of description, a side that is of the device body 11 and on which the display screen is located on is referred to as a front side, and a side that is of the device body 11 and on which the display screen faces away from the device body 11 is referred to as a rear side of the device body 11. In addition, a part that is of the flexible fixing strip 12 and that is on a same side as the display screen on the device body 11 is referred to as a front side of the flexible fixing strip 12, and a side that faces away from the front side of the flexible fixing strip 12 is referred to as a rear side of the flexible fixing strip 12. An electrode disposed on the device body 11 may be located on the rear side of the device body 11, or may be located on the front side of the device body 11. An electrode disposed on the flexible fixing strip 12 may be located on the front side of the flexible fixing strip 12, or may be located on the rear side of the flexible fixing strip.

It may be understood that a shape and a material of the flexible fixing strip 12 are not limited in this embodiment. For example, the flexible fixing strip 12 may be implemented by using a flexible strip, and two ends of the flexible strip are respectively connected to two ends of the device body 11. The flexible fixing strip 12 may alternatively be implemented by using two flexible strips. One end of each of the two flexible strips is separately connected to one end of two ends of the device body 11, and the other end of each of the two flexible strips implements detachable connection by using a connection structure, for example, locking or a threaded connection. For example, the flexible fixing strip 12 may be understood as a strip that binds the device body 11 of the wearable device 100 to the user body, so that the user carries the wearable device 100. For example, when the wearable device 100 is a watch or a band, the flexible fixing strip 12 may be referred to as a watch band.

For example, still refer to FIG. 1. When two groups of electrodes are disposed on the wearable device 100, and each group of electrodes includes one electrode 14, one group of electrodes may be disposed on the device body 11, and the other group of electrodes may be disposed on the flexible fixing strip 12. An electrode on the device body 11 is located on the rear side of the device body 11, and an electrode on the flexible fixing strip 12 is located on the rear side of the flexible fixing strip 12. As shown in FIG. 2, when two groups of electrodes are disposed on the wearable device 100, and each group of electrodes includes one electrode 14, one group of electrodes may be disposed on the device body 11, and the other group of electrodes may be disposed on the flexible fixing strip 12. An electrode on the device body 11 is located on the rear side of the device body 11, and an electrode on the flexible fixing strip 12 is located on the front side of the flexible fixing strip 12. For example, in FIG. 1 and FIG. 2, a distance (the distance is a distance when the flexible fixing strip 12 is in an unfolded state) between a group of electrodes located on the device body 11 and a group of electrodes located on the flexible fixing strip 12 may be greater than a preset distance, for example, greater than 15 centimeters.

As shown in FIG. 3, when two groups of electrodes are disposed on the wearable device 100, and each group of electrodes includes one electrode 14, both the two groups of electrodes may be disposed on the flexible fixing strip 12, and a distance (the distance is a distance when the flexible fixing strip 12 is in an unfolded state) between the two groups of electrodes is greater than a preset distance, for example, greater than 15 centimeters. Both the two groups of electrodes may be located on the rear side of the flexible fixing strip 12. As shown in FIG. 4, when two groups of electrodes are disposed on the wearable device 100, and each group of electrodes includes one electrode 14, both the two groups of electrodes may be disposed on the flexible fixing strip 12, and a distance (the distance is a distance when the flexible fixing strip 12 is in an unfolded state) between the two groups of electrodes is greater than a preset distance, for example, greater than 15 centimeters. One group of electrodes may be located on the rear side of the flexible fixing strip 12, and the other group of electrodes may be located on the front side of the flexible fixing strip 12. When the flexible fixing strip 12 is formed by a flexible strip 121 and a flexible strip 122, one group of electrodes may be disposed at an end that is of the flexible strip 121 and that is away from the device body 11, and the other group of electrodes 14 may be disposed at an end that is of the flexible strip 122 and that is away from the device body 11. In an example, the flexible strip 121 may be referred to as a first flexible strip, and the flexible strip 122 may be referred to as a second flexible strip.

For example, as shown in FIG. 5, when two groups of electrodes are disposed on the wearable device 100, and each group of electrodes includes two electrodes 14, one group of electrodes may be disposed on the device body 11, and the other group of electrodes may be disposed on the flexible fixing strip 12. Electrodes on the device body 11 are located on the rear side of the device body 11, and electrodes on the flexible fixing strip 12 are located on the rear side of the flexible fixing strip 12. As shown in FIG. 6, when two groups of electrodes are disposed on the wearable device 100, and each group of electrodes includes two electrodes 14, one group of electrodes may be disposed on the device body 11, and the other group of electrodes may be disposed on the flexible fixing strip 12. Electrodes on the device body 11 are located on the rear side of the device body 11, and electrodes on the flexible fixing strip 12 are located on the front side of the flexible fixing strip 12. For example, a shape of an electrode located on the device body 11 and/or the flexible fixing strip 12 may be one or more of a circle, an ellipse, a semicircle, an arc, or a rectangle. For example, the electrodes located on the device body 11 may be symmetrically arranged along a center line of the device body 11 in a direction (for example, in an extension direction from one end of the flexible fixing strip 12 to the other end of the flexible fixing strip 12), and there is a preset distance between different electrodes. It may be understood that the electrodes located on the device body 11 may be arranged in another manner. This is not limited herein. For example, the electrodes located on the flexible fixing strip 12 may be spaced apart on the flexible fixing strip 12, and each electrode on the flexible fixing strip 12 is at a specific distance from the device body 11. Still refer to FIG. 5. The electrodes on the flexible fixing strip 12 may be sequentially arranged in an x direction, or may be sequentially arranged in a direction perpendicular to the x direction, or may be sequentially arranged in another direction. This is not limited herein. For example, in FIG. 5 and FIG. 6, a distance (the distance is a distance when the flexible fixing strip 12 is in an unfolded state) between a group of electrodes located on the device body 11 and a group of electrodes located on the flexible fixing strip 12 may be greater than a preset distance, for example, greater than 15 centimeters.

As shown in FIG. 7, when two groups of electrodes are disposed on the wearable device 100, and each group of electrodes includes two electrodes 14, both the two groups of electrodes may be disposed on the flexible fixing strip 12, and a distance (the distance is a distance when the flexible fixing strip 12 is in an unfolded state) between the two groups of electrodes is greater than a preset distance, for example, greater than 15 centimeters. Both the two groups of electrodes are located on the rear side of the flexible fixing strip 12, one group of electrodes may be located at one end of the flexible fixing strip 12, and the other group of electrodes may be located at the other end of the flexible fixing strip 12. As shown in FIG. 8, when two groups of electrodes are disposed on the wearable device 100, and each group of electrodes includes two electrodes 14, both the two groups of electrodes may be disposed on the flexible fixing strip 12, and a distance (the distance is a distance when the flexible fixing strip 12 is in an unfolded state) between the two groups of electrodes is greater than a preset distance, for example, greater than 15 centimeters. One group of electrodes may be located at one end of the rear side of the flexible fixing strip 12, and the other group of electrodes may be located at one end of the front side of the flexible fixing strip 12. When the flexible fixing strip 12 is formed by a flexible strip 121 and a flexible strip 122, one group of electrodes may be disposed at an end that is of the flexible strip 121 and that is away from the device body 11, and the other group of electrodes may be disposed at an end that is of the flexible strip 122 and that is away from the device body 11. For arrangement manners of the electrodes on the flexible strip 121 and the flexible strip 122, refer to the foregoing descriptions about the arrangement manners of the electrodes in FIG. 5. Details are not described herein again.

It may be understood that, when three or more groups of electrodes are disposed on the wearable device 100, for an arrangement manner of a plurality of groups of electrodes on the wearable device 100, refer to the foregoing described arrangement manners of the two groups of electrodes. Details are not described herein again.

For example, when at least one group of electrodes is disposed on the flexible fixing strip 12, an electrode 14 in the at least one group of electrodes may be pasted on an outer side of the flexible fixing strip 12. When the at least one group of electrodes is disposed on the device body 11, an electrode 14 in the at least one group of electrodes may be coated on the device body 11. For example, the electrode 14 may be fixed to a surface of the device body 11 by coating the device body 11.

For example, when the flexible fixing strip 12 is connected to the device body 11 in an undetachable manner, the flexible fixing strip 12 and the device body 11 may be designed in an integrated manner. This is not limited herein. In this case, the electrode on the flexible fixing strip 12 may be directly connected to the processor 13. For example, the device body 11 may be provided with a cable channel a (not shown in the figure), and the flexible fixing strip 12 may be provided with a cable channel b (not shown in the figure). The cable channel a and the cable channel b are communicated with each other, and the cable channels a and b are both configured to accommodate a cable configured to connect the processor 13 to the electrode on the flexible fixing strip 12, to implement an electrical connection between the processor 13 and the electrode on the flexible fixing strip 12. In an example, the cable channel a may be referred to as a second cable channel, the cable channel b may be referred to as a first cable channel, and the cable configured to connect the processor 13 to the electrode on the flexible fixing strip 12 may be referred to as a first cable.

For example, when the flexible fixing strip 12 is detachably connected to the device body 11, as shown in FIG. 9, a locking piece 1231, a locking piece 1232, an elastic component 1233, and a pushing member 1234 may be disposed in a fixing strip body 123 at an end that is of the flexible fixing strip 12 and that is connected to the device body 11. In an X direction shown in FIG. 9, the locking piece 1231, the elastic component 1233, the pushing member 1234, and the locking piece 1232 are arranged sequentially. When the elastic component 1233 is in a natural state, both the locking piece 1231 and the locking piece 1232 are partially located in the fixing strip body 123, and partially located outside the fixing strip body 123. When external force is applied to the pushing member 1234 and the elastic component 1233 is pressed, the pushing member 1234 may drive the locking piece 1232 to move towards the locking piece 1231, so that a part of the locking piece 1232 located outside the fixing strip body 123 may move toward an interior of the fixing strip body 123. When the electrode 14 is disposed on the flexible fixing strip 12, the electrode 14 may be connected to the locking piece 1231 and/or the locking piece 1232 by using a cable. The locking piece 1231 and/or the locking piece 1232 may be a conductor. For example, the elastic component 1233 may be a spring. For example, when the electrode is disposed on the flexible fixing strip 12, the flexible fixing strip 12 may be provided with a cable channel c (not shown in the figure), and the cable channel c may accommodate the cable configured to connect the electrode to the locking piece. In an example, the locking piece 1231 may be referred to as a first locking piece, and the locking piece 1232 may be referred to as a second locking piece.

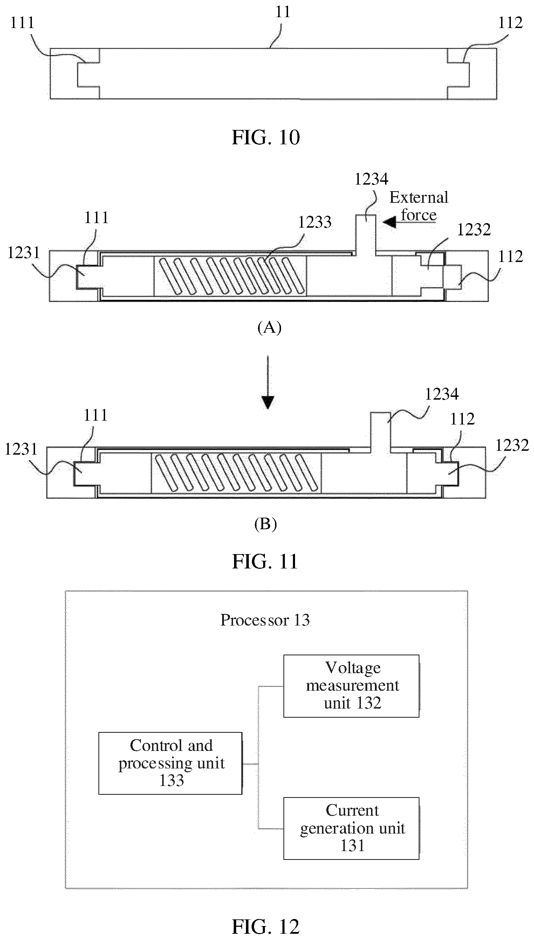

As shown in FIG. 10, the device body 11 may be provided with a slot 111 to which the locking piece 1231 is fitted and a slot 112 to which the locking piece 1232 is fitted. The slot 111 may be connected to the processor 13 by using a cable, and the slot 112 may also be connected to the processor 13 by using a cable. In addition, a conductor may be disposed in both the slot 111 and the slot 112, so that the processor 13 is electrically connected to the electrode 14. The device body 11 may be provided with a cable channel d (not shown in the figure), and the cable channel d may accommodate a cable configured to connect the processor 13 to the slot. In an example, the slot 111 may be referred to as a first slot, and the slot 112 may be referred to as a second slot.

As shown in (A) of FIG. 11, to mount the flexible fixing strip 12 on the device body 11, the locking piece 1231 may be first placed in the slot 111, and the pushing member 1234 is controlled to move towards the locking piece 1231, so that the locking piece 1232 moves towards the locking piece 1231, so that the fixing strip body 123 may be located between the slot 111 and the slot 112. Then, as shown in (B) of FIG. 11, external force may be stopped being applied to the pushing member 1234. By using the elastic component 1233, the pushing member 1234 pushes the locking piece 1232 into the slot 112. In this way, the flexible fixing strip 12 is mounted on the device body 11. It may be understood that, when the flexible fixing strip 12 is removed, steps opposite to the steps of mounting the flexible fixing strip 12 may be used. Details are not described herein again. For example, the locking piece 1231 may be electrically connected to the slot 111 by using a pogo pin (pogo pin), an elastic piece, or the like, and the locking piece 1232 may also be electrically connected to the slot 112 by using a pogo pin (pogo pin), an elastic piece, or the like.

For example, as shown in FIG. 12, a current generation unit 131, a voltage measurement unit 132, and a control and processing unit 133 may be disposed in the processor 13 in the wearable device 100. The current generation unit 131 may generate a current under control of the control and processing unit 133, for example, generate a current of 50 micro-amps ($\mu$A) or 100 micro-amps ($\mu$A). The current generation unit 131 may be connected to an electrode in the wearable device 100. In addition, the current generation unit 131 may also generate currents of different frequencies under control of the control and processing unit 133, for example, generate a current of 50 kilohertz (kHz) or 250 kilohertz (kHz). The voltage measurement unit 132 may measure a voltage. The voltage measurement unit 132 may be connected to the electrode in the wearable device 100. The control and processing unit 133 may control the current generation unit 131 to generate a current. Based on the voltage value measured by the voltage measurement unit 132 and the current value generated by the current generation unit 131, an impedance of a human body may also be determined. For example, the impedance of a human body may be a ratio of the voltage value measured by the voltage measurement unit 132 to the current value generated by the current generation unit 131. For example, current values of currents at different frequencies may be the same, voltage values corresponding to currents at different frequencies may be the same, or may be different, and currents at different frequencies are generated at different moments.

For example, after a user starts measurement, the current generation unit 131 may apply a voltage between one electrode in a group of electrodes and one electrode in another group of electrodes. After the user enables both the electrodes on the wearable device 100 to get in contact with the body, the two electrodes are electrically connected to each other, and a current is generated. In this case, the current generation unit 131 may adjust a value of the voltage, so that the current reaches a preset current value (for example, 50 micro-amps), and the current value is controlled to maintain continuously in a process of the measurement. For example, when the current value reaches the preset current value, the voltage may be kept unchanged, to keep the current value unchanged. For example, after the user starts the measurement, the current generation unit 131 may apply a preset voltage between one electrode in a group of electrodes and one electrode in another group of electrodes. After the user enables both the electrodes on the wearable device 100 to get in contact with the body, the two electrodes are electrically connected to each other, and a current is generated. In this case, the current generation unit 131 may measure the current value. Then, an impedance value may be obtained based on the current value and the preset voltage value. In addition, to improve measurement accuracy, the voltage measurement unit 132 may also measure the voltage, and an impedance value is determined by using the measured voltage value and the measured current value. It may be understood that, in this embodiment, in addition to all of the current generation unit 131, the voltage measurement unit 132, and the control and processing unit 133 being integrated into the processor 13, any one or two of the current generation unit 131, the voltage measurement unit 132, and the control and processing unit 133 may alternatively be integrated into the processor 13, and the rest may be disposed independently. In addition, the current generation unit 131, the voltage measurement unit 132, and the control and processing unit 133 may alternatively be separately disposed. This is not limited herein.

For example, when a plurality of groups of electrodes on the wearable device 100 are all disposed on a same side of the wearable device 100 (as shown in cases in FIG. 1, FIG. 3, FIG. 5, and FIG. 7), when the user uses the wearable device 100 to measure liver fat, the user may place the wearable device 100 on skin corresponding to an area in which the user's liver body is located. During measurement, the user may place some electrodes of the wearable device 100 in an area in which one end of the body liver is located, and place some electrodes in an area in which the other end of the body liver is located. For example, when an electrode is disposed on the device body 11 of the wearable device 100, and an electrode is disposed on the flexible fixing strip 12, as shown in (A) of FIG. 13, the electrode on the device body 11 may be attached to a xiphoid process position (for example, an area A in the figure) on the user body, and the electrode on the flexible fixing strip 12 may be attached to a position (for example, an area B in the figure) below a rib on a right side of the user body. Alternatively, as shown in (B) of FIG. 13, the electrode on the flexible fixing strip 12 may be attached to a xiphoid process position (for example, an area A in the figure) on the user body, and the electrode on the device body 11 may be attached to a position (for example, an area B in the figure) below a rib on a right side of the user body. For example, when each electrode on the wearable device 100 is disposed on the flexible fixing strip 12, as shown in FIG. 14, an electrode on a side of the flexible fixing strip 12 is attached to a xiphoid process position (for example, an area A in the figure) on the user body, and an electrode on the other side of the flexible fixing strip 12 is attached to a position below a rib on a right side of the user body (for example, an area B in the figure). It may be understood that, in this manner, when the wearable device 100 is worn on the user body (for example, a wrist), the user may remove the wearable device 100 from the user body (for example, the wrist) to perform the measurement. In an example, the xiphoid process position on the user body (for example, the area A in FIG. 13) may be referred to as a first position, and the position below a rib on a right side of the user body (for example, the area B in FIG. 13) may be referred to as a second position.

Further, after the user places the wearable device 100 in the area in which the user's liver body is located, as shown in FIG. 13 or FIG. 14, the electrode in contact with the area A of the user body is electrically connected to the electrode in contact with the area B of the user body. Because a skin impedance is large, and a liver impedance is small, the current generated by the current generation unit 131 in the wearable device 100 first flows through a loop with a small impedance. In other words, the current flows through the liver instead of a skin surface layer. In addition, in a process in which the current flows through the liver, the voltage measurement unit 132 in the wearable device 100 may measure a voltage value between the electrode located in the area A and the electrode located in the area B. Then, the liver impedance may be obtained based on the voltage value measured by the voltage measurement unit 132 and the current value generated by the current generation unit 131.

For example, when electrodes on the wearable device 100 are disposed on different sides of the wearable device 100 (as shown in FIG. 2, FIG. 4, FIG. 6, and FIG. 8), when the user uses the wearable device 100 to measure liver fat, the user may not need to remove the wearable device 100 from the user body (for example, a wrist). For details, refer to the following descriptions.

For example, a part of electrode on the wearable device 100 may be located on the device body 11, the part of electrode is in contact with the wrist of the user, and the other part of electrode is located on the flexible fixing strip 12 on the wearable device 100 and is located on a side away from the wrist of the user, that is, a case shown in FIG. 6. As shown in FIG. 15, an electrode on the device body 11 is in contact with a wrist (that is, an area C in the figure) of the user. The user may first attach an electrode on the flexible fixing strip 12 to a xiphoid process position (for example, an area D in the figure) on the user body, and then attach the electrode on the flexible fixing strip 12 to a position below a rib on a right side of the user body (for example, an area E in the figure). Finally, a liver impedance is obtained based on data from two measurements. Still refer to FIG. 15. When the user attaches the electrode on the flexible fixing strip 12 to the xiphoid process position (for example, the area D in the figure) on the user body, the area D may be electrically connected to the area C, a current generated by the current generation unit 131 in the wearable device 100 may flow through the area D and the area C and an area (for example, an arm, a chest, and the like) between the area C and the area D, and the voltage measurement unit 132 in the wearable device 100 may measure a voltage value between the area D and the area C, to obtain an impedance between the area D and the area C. Similarly, still refer to FIG. 15. When the user attaches the electrode on the flexible fixing strip 12 to the position below a rib on a right side of the user body (for example, the area E in the figure), the area E may be electrically connected to the area C, a current generated by the current generation unit 131 in the wearable device 100 may flow through the area E and the area C and an area (for example, an arm, a chest, a liver, and the like) between the area C and the area E, and the voltage measurement unit 132 in the wearable device 100 may measure a voltage value between the area E and the area C, to obtain an impedance between the area E and the area C. Further, the impedance of the liver may be obtained based on the impedance between the area D and the area C and the impedance between the area E and the area C.

In a process of obtaining the impedance of the liver, an arm of the user may be equivalent to a resistor, an upper left chest or an upper right chest of the user may be equivalent to a resistor, and the user's liver may be equivalent to a resistor. For example, when the wearable device 100 is worn on a left hand of the user, as shown in FIG. 16, the left arm of the user may be equivalent to a resistor R1, the upper left chest of the user may be equivalent to a resistor R2, and the user's liver may be equivalent to a resistor R3. Still refer to FIG. 15, with reference to FIG. 16. When the user attaches the electrode on the flexible fixing strip 12 to the xiphoid process position (for example, the area D in the figure) on the user body, a resistance R01=R1+R2 is measured. When the user attaches the electrode on the flexible fixing strip 12 to the position (for example, the area E in the figure) below a rib on a right side of the user body, a resistance R02=R1+R2+R3 is measured. After the resistances R01 and R02 are obtained, the impedance of the liver R3=R02-R01 may be learned. In an example, the xiphoid process position on the user body (for example, the area D in FIG. 16) may be referred to as a first position, and the position below a rib on a right side of the user body (for example, the area E in FIG. 16) may be referred to as a second position. In an example, R01 may be referred to as a first physiological parameter, R02 may be referred to as a second physiological parameter, and R03 may be referred to as a third physiological parameter.

For example, in a process of measuring the impedance of the liver, when the impedance of the liver is measured by using two electrodes (that is, measured by using two groups of electrodes, and each group of electrodes includes one electrode), as shown in (A) of FIG. 17, a current loop may be formed between an electrode a, the current generation unit 131, and an electrode b on the wearable device 100 and a human body Q. In addition, a voltage measurement loop may be formed between the electrode a, the voltage measurement unit 132, and the electrode b on the wearable device 100 and the human body Q. During measurement, the control and processing unit 133 in the processor 13 of the wearable device 100 may control the current generation unit 131 to generate a current I, and the current I flows through the formed current loop. In addition, the voltage measurement unit 132 may measure a voltage U (that is, a voltage of the human body) between the electrodes a and b. Finally, an impedance of the human body Q is obtained based on the voltage U and the current I.

When the impedance of the liver is measured by using four electrodes (that is, measured by using two groups of electrodes, and each group of electrodes includes two electrodes), for example, the four electrodes are electrodes a, b, c, and d, the electrodes a and c may be located in a same area on the wearable device 100, and the electrodes b and d may be located in a same area on the wearable device 100. For example, as shown in FIG. 6, the electrodes a and c may be electrodes located on the device body 11 of the wearable device 100, and the electrodes b and d may be electrodes located on the flexible fixing strip 12 of the wearable device 100. As shown in (B) of FIG. 17, a current loop may be formed between the electrode a, the current generation unit 131, and the electrode b on the wearable device 100 and a human body Q. In addition, a voltage measurement loop may be formed between the electrode c, the voltage measurement unit 132, and the electrode d on the wearable device 100 and the human body Q. During measurement, the control and processing unit 133 in the processor 13 of the wearable device 100 may control the current generation unit 131 to generate a current I, and the current I flows through the formed current loop. In addition, the voltage measurement unit 132 may measure a voltage U (that is, a voltage of the human body) between the electrodes c and d. Finally, an impedance of the human body Q is obtained based on the voltage U and the current I. It may be understood that, for a measurement method in which more electrodes are used for measuring the impedance of the liver, refer to the measurement method using two or four electrodes, and details are not described herein again.

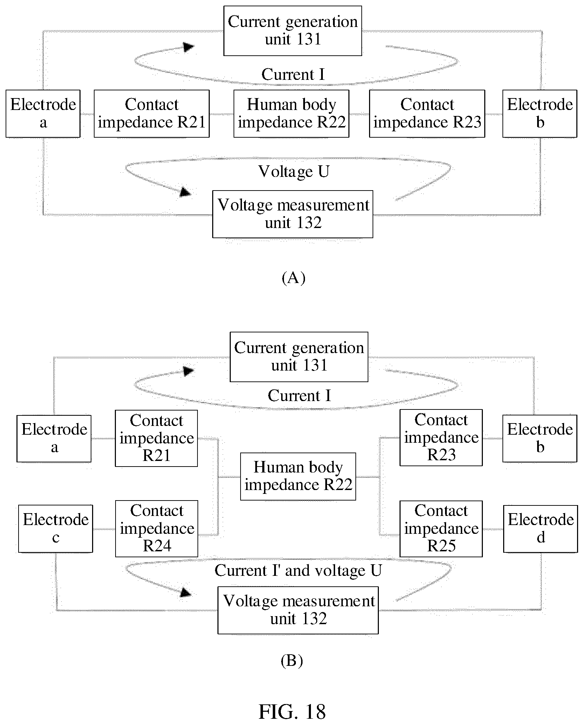

It may be understood that when the electrode on the wearable device 100 is in contact with the user body, there is a contact impedance between the electrode and the body user. When the impedance of the liver is measured by using two electrodes, as shown in (A) of FIG. 18, if a current generated by the current generation unit 131 is I, contact impedances are R21 and R23, and an impedance of the human body is R22, a voltage measured by the voltage measurement unit 133 is U=IxR21+IxR22+IxR23. Therefore, in this case, a measured impedance is actually U/I=R21+R22+R23. It can be seen that in this case, the obtained impedance is two more contact impedance values than the real impedance of the human body. Generally, the impedance of the human body is about 300 S2 to 1000 $\Omega$, and the contact impedance may be generally greater than 30 Q. Therefore, the contact impedance has a great impact on a measurement result. In other words, precision of the measured impedance of the liver obtained by using the two electrodes is relatively low.

When the impedance of the liver is measured by using four electrodes (that is, measured by using two groups of electrodes, and each group of electrodes includes two electrodes), as shown in (B) of FIG. 18, if a current generated by the current generation unit 131 is I, contact impedances are R21, R23, R24, and R25, and an impedance of the human body is R22. In a voltage measurement loop, the impedance R22 of the human body and the two contact resistances R24 and R25 are connected in series. When measuring the voltage, the voltage measurement unit 132 may generate a current I' that is approximately 0. In this case, a current flowing through the human body is I+I', and the voltage measured by the voltage measurement unit 132 is U=I'xR24+(I+I')xR22+I'xR25. Because I' is approximately 0, U=IxR22 is obtained. Therefore, an obtained impedance of the human body is U/I=R22. It can be seen that the obtained impedance of the human body may reflect the real impedance of the human body. In other words, precision of the measured impedance of the liver obtained by using the four electrodes is relatively high.

After the impedance of the liver is measured, fat content of the liver may be obtained based on the impedance of the liver, to evaluate a liver risk level.

For example, the fat content of the liver may be calculated by using the following formula. The formula (referred to as "Formula 1") is:

$$M = \alpha_1 R + \frac{\alpha_2}{R} + \alpha_3 \quad \text{Formula 1}$$

$M$ is the fat content of the liver; $R$ is the impedance of the liver; and $\alpha_1$, $\alpha_2$, and $\alpha_3$ are preset coefficients, and may be obtained through experiment.

For example, a body parameter such as a height of the user may also be combined in calculation on the fat content of the liver. In this case, the fat content of the liver may also be calculated by using the following formula. The formula (referred to as "Formula 2") is:

$$M = \alpha_1 R + \frac{\alpha_2}{R} + \alpha_3 H + \alpha_4 \quad \text{Formula 2}$$

$M$ is the fat content of the liver; $R$ is the impedance of the liver; $H$ is the height; and $\alpha_1$, $\alpha_2$, $\alpha_3$ and $\alpha_4$ are preset coefficients, and may be obtained through experiment.

For example, when the impedance of the liver is measured by using currents of a plurality of different frequencies, the fat content of the liver may be calculated by using the following formula. The formula (referred to as "Formula 3") is:

$$M = \alpha_1 R_1 + \frac{\alpha_2}{R_1} + \alpha_3 R_2 + \frac{\alpha_4}{R_2} + \alpha_5 \quad \text{Formula 3}$$

$M$ is the fat content of the liver; $R_1$ and $R_2$ are impedances of the liver measured under currents of different frequencies; and $\alpha_1$, $\alpha_2$, $\alpha_3$, $\alpha_4$, and $\alpha_5$ are preset coefficients, and may be obtained through experiment. It can be understood that, when a quantity of impedances of a liver measured under currents of different frequencies is greater than or equal to three, "$\alpha_n R_n + \frac{\alpha_{n+1}}{R_n}$" may be added to Formula 3. $R_n$ is an $n^{\text{th}}$ impedance of the liver, and $\alpha_n$ and $\alpha_{n+1}$ are preset coefficients corresponding to $R_n$, and may be obtained through experiment.

For example, when the impedance of the liver is measured by using currents of a plurality of different frequencies, and the fat content of the liver is calculated with reference to a body parameter such as a height of the user, the fat content of the liver may also be calculated by using the following formula. The formula (referred to as "Formula 4") is:

$$M = \alpha_1 R_1 + \frac{\alpha_2}{R_1} + \alpha_3 R_2 + \frac{\alpha_4}{R_2} + \alpha_5 H + \alpha_6 \quad \text{Formula 4}$$

$M$ is the fat content of the liver; $R_1$ and $R_2$ are impedances of the liver measured under currents of different frequencies; H is the height; and $\alpha_1$, $\alpha_2$, $\alpha_3$, $\alpha_4$, $\alpha_5$, and $\alpha_6$ are preset coefficients, and may be obtained through experiment. It can be understood that, when a quantity of impedances of a liver measured under currents of different frequencies is greater than or equal to three, "$\alpha_n R_n + \frac{\alpha_{n+1}}{R_n}$" may be added to Formula 4. $R_n$ is an $n^{\text{th}}$ impedance of the liver, and $\alpha_n$ and $\alpha_{n+1}$ are preset coefficients corresponding to $R_n$, and may be obtained through experiment.

After the fat content of the liver is obtained, the risk level of the user's liver may be determined based on a correspondence between the fat content of the liver and the risk level of the liver. For example, a preset correspondence between the fat content of the liver and the risk level of the liver may be shown in Table 1. When it is determined that fat content of the liver is "5", it can be learned from Table 1 that a risk level of the liver in this case is "suspicious risk".

**Table 1**

| Liver fat content | Liver risk level |
| --- | --- |
| 0 to 4 | Normal |
| 4 to 7 | Suspected risk |
| 7 to 10 | Medium to high risk |

The foregoing describes the wearable device 100 and a related measurement principle in the technical solutions of this application. The following separately describes a process of testing a risk level of a liver by using examples in which electrodes of the wearable device 100 are disposed on a same side and different sides of the wearable device 100.

(1) All electrodes of the wearable device 100 are disposed on a same side of the wearable device 100 (a case shown in FIG. 1, FIG. 3, FIG. 5, or FIG. 7).

When all electrodes of the wearable device 100 are disposed on a same side of the wearable device 100 (a case shown in FIG. 1, FIG. 3, FIG. 5, or FIG. 7), the measurement manners described in FIG. 13 or FIG. 14 may be used. In this case, after the user attaches all the electrodes on the wearable device 100 to a specific position on the user body, the testing may be performed.

Specifically, an application (for example, Huawei Health) related to liver fat measurement may be installed on the wearable device 100. As shown in (A) of FIG. 19, after the user opens the application related to liver fat measurement on the wearable device 100 and starts the liver fat measurement, the wearable device 100 may display personal information of the user, such as a name and a height. The user may modify the personal information of the user on the display interface. In (A) of FIG. 19, the user may tape an "OK" button in an area a1 to go to a next step. Then, as shown in (B) of FIG. 19, the wearable device 100 may display guide information. The guide information is mainly used for guiding the user to attach an electrode on the wearable device 100 to a specific position on the user body, for example, attach the electrode to a xiphoid process position on the user body or a position below a right rib of the user body. The guide information may be text information, or may be voice information, or may be graphic information, or may be any combination of two or three of the text information, the voice information, and the graphic information. It may be understood that, when selecting a button on the wearable device 100, the user may select the button by tapping, by a voice, or by using a gesture. This may be specifically determined according to an actual situation, and is not limited herein. In addition, after the wearable device 100 displays the guide information (that is, displays an interface shown in (B) of FIG. 19), the wearable device 100 may apply a current required for the measurement. In addition, before displaying the guide information, the wearable device 100 may also generate a current required for the measurement. This is not limited herein. In an example, in (A) of FIG. 19, the operation in which the user may tap the "OK" button in the area a1 may be referred to as a first operation. In an example, the interface shown in (B) of FIG. 19 may be referred to as a first interface.

In a measurement process, the wearable device 100 may display an interface shown in (C) of FIG. 19. Then, after measuring the liver fat content of the user, the wearable device 100 may prompt, by using a voice, the user that the measurement is completed, or prompt, by using a combination of a voice and a text, the user that the measurement is completed, and display a test result, that is, display an interface shown in (D) of FIG. 19. In addition, after displaying the interface shown in (D) of FIG. 19, the wearable device 100 may also display a related result interpretation, that is, display an interface shown in (E) of FIG. 19. For example, if a risk of fatty liver is high, a result interpretation can be as follows: You have a high risk of fatty liver. To ensure your health, please strictly control your diet, especially oil and alcohol intake, and it is recommended that you perform aerobic exercise for at least one hour every day. If a risk of fatty liver is moderate, a result interpretation can be as follows: You have a moderate risk of fatty liver. To ensure your health, please control your diet, especially oil and alcohol intake, and it is recommended that you perform aerobic exercise every day. When a risk of fatty liver is low, a result interpretation can be as follows: You have a low risk of fatty liver. To ensure your health, please eat properly, exercise properly, and control alcohol intake. In addition, in addition to displaying the test result, the wearable device 100 may also broadcast the test result or the like by using a voice.

It may be understood that, on the interface shown in (C) of FIG. 19, the wearable device 100 needs to measure an impedance of the liver, and obtain the fat content of the liver based on the impedance of the liver, to obtain a risk level of the liver. When the impedance of the liver is measured, the impedance of the liver may be compared with a preset impedance range, to determine whether the measured impedance of the liver is reasonable. When the impedance of the liver falls within the preset impedance range, the measured impedance of the liver is reasonable; otherwise, the measured impedance of the liver is unreasonable. Further, when it is determined that the measured impedance of the liver is unreasonable, the user may be prompted to perform measurement again. For example, the user may be prompted to perform the measurement again by using a voice. After prompting the user, the wearable device 100 may return to display the interface shown in (B) of FIG. 19.

It may be understood that in a measurement process, the wearable device 100 may display different interfaces at different measurement stages, to prompt the user. For example, after the user starts testing the risk level of the liver, the wearable device 100 may display an interface shown in (A) of FIG. 20, to prompt the user to start fatty liver screening. Then, the wearable device 100 may display an interface shown in (B) of FIG. 20, to visually prompt the user to place the wearable device 100 at a preset position or area on the user body. A placement prompt may be the same as the guide information in (B) of FIG. 19. Then, after the user places the wearable device 100 at the preset position or area, the wearable device 100 may display an interface shown in (C) of FIG. 20, to prompt the user that liver impedance measurement is being performed. After the liver impedance is measured, the wearable device 100 may display an interface shown in (D) of FIG. 20, to prompt the user that the risk level testing of the liver is being performed. After the risk level of the liver is tested, the wearable device 100 may display an interface shown in (E) of FIG. 20, to display the risk level of the liver to the user. Then, the wearable device 100 may display, after a preset time (for example, 3s), an interface shown in (F) of FIG. 20, to present a testing result interpretation to the user. The result interpretation may be the same as the result interpretation in (E) of FIG. 19. In addition, to switch from (E) of FIG. 20 to (F) of FIG. 20, in addition to automatic switching, the user may also perform a manual operation for switching. For example, in (E) of FIG. 20, when the user performs a sliding operation (for example, slide up, slide down, slide left, or slide right) in a direction on the device body 11 of the wearable device 100, the wearable device 100 switches to (F) of FIG. 20. It should be noted that the display interfaces shown in FIG. 20 are merely examples for description, and do not constitute a limitation on this solution. The display interfaces may be selected according to a requirement, or may be redrawn according to a requirement. This is not limited herein.

(2) Electrodes of the wearable device 100 are disposed on different sides of the wearable device 100 (a case shown in FIG. 2, FIG. 4, FIG. 6, or FIG. 8).

When electrodes of the wearable device 100 are disposed on different sides of the wearable device 100 (a case shown in FIG. 2, FIG. 4, FIG. 6, or FIG. 8), the measurement manner described in FIG. 15 may be used. In this case, after the user enables a part of the electrodes on the wearable device 100 to get in contact with a wearing position (for example, a wrist) of the user, and places the other part of the electrodes to a specific position on the user body, measurement may be performed.

Specifically, an application (for example, Huawei Health) related to liver fat measurement may be installed on the wearable device 100. As shown in (A) of FIG. 21, after the user opens the application related to liver fat measurement on the wearable device 100 and starts the liver fat measurement, the wearable device 100 may display personal information of the user, such as a name and a height. The user may modify the personal information of the user on the display interface. In (A) of FIG. 21, the user may tape an "OK" button in an area a1 to go to a next step. Then, as shown in (B) of FIG. 21, the wearable device 100 may display guide information b. The guide information b is mainly used for guiding the user to attach an electrode on the wearable device 100 to a specific position on the user body, for example, attach the electrode to a xiphoid process position on the user body or a position below a right rib of the user body. The guide information b may be text information, or may be voice information, or may be graphic information, or may be any combination of two or three of the text information, the voice information, and the graphic information. It may be understood that, when selecting a button on the wearable device 100, the user may select the button by tapping, by a voice, or by using a gesture. This may be specifically determined according to an actual situation, and is not limited herein. In addition, after the wearable device 100 displays the guide information b (that is, displays an interface shown in (B) of FIG. 21), the wearable device 100 may generate a current required for the measurement. In addition, before displaying the guide information b, the wearable device 100 may also generate a current required for the measurement. This is not limited herein. In an example, in (A) of FIG. 21, the operation in which the user may tap the "OK" button in the area a1 may be referred to as a first operation. In an example, the interface shown in (B) of FIG. 21 may be referred to as a second interface.

In a measurement process, the wearable device 100 may display an interface shown in (C) of FIG. 21. Then, after measuring a human body impedance R of the user, the wearable device 100 may prompt the user that the measurement is completed, for example, by using a voice prompt and/or a vibration prompt, and display an interface shown in (D) of FIG. 21, that is, display guide information c. The guide information c is mainly used for guiding the user to attach an electrode of the wearable device 100 to a specific position on the user body, for example, attach the electrode to a xiphoid process position on the user body or a position below a right rib of the user body. The placement position of the electrode guided by the guide information c is different from the placement position of the electrode guided by the guide information b. For example, when the placement position of the electrode guided by the guide information b is the xiphoid process position on the user body, the placement position of the electrode guided by the guide information c is the position below a right rib of the user body. In an example, the interface shown in (D) of FIG. 21 may be referred to as a third interface.

Then, the wearable device 100 may display an interface shown in (E) of FIG. 21. After the wearable device 100 measures the human body impedance R of the user again, the wearable device 100 may determine liver fat content based on impedance values from two measurements. After measuring the liver fat content of the user, the wearable device 100 may prompt, by using a voice, the user that the measurement is completed, or prompt, by using a combination of a voice and a text, the user that the measurement is completed, and display a test result, that is, display an interface shown in (F) of FIG. 21. In addition, after displaying the interface shown in (F) of FIG. 21, the wearable device 100 may also display a related result interpretation, that is, display an interface shown in (G) of FIG. 21. For example, if a risk of fatty liver is high, a result interpretation can be as follows: You have a high risk of fatty liver. To ensure your health, please strictly control your diet, especially oil and alcohol intake, and it is recommended that you perform aerobic exercise for at least one hour every day. If a risk of fatty liver is moderate, a result interpretation can be as follows: You have a moderate risk of fatty liver. To ensure your health, please control your diet, especially oil and alcohol intake, and it is recommended that you perform aerobic exercise every day. When a risk of fatty liver is low, a result interpretation can be as follows: You have a low risk of fatty liver. To ensure your health, please eat properly, exercise properly, and control alcohol intake. In addition, in addition to displaying the test result, the wearable device 100 may also broadcast the test result or the like by using a voice.

For example, when the impedance of the liver is measured, the impedance of the liver may be compared with a preset impedance range, to determine whether the measured impedance of the liver is reasonable. When the impedance of the liver falls within the preset impedance range, the measured impedance of the liver is reasonable; otherwise, the measured impedance of the liver is unreasonable. Further, when it is determined that the measured impedance of the liver is unreasonable, the user may be prompted to perform measurement again. For example, the user may be prompted to perform the measurement again by using a voice. After prompting the user, the wearable device 100 may return to display the interface shown in (B) of FIG. 21.

For example, in the interfaces shown in (C) and (E) of FIG. 21, the wearable device 100 may both check the measured body impedances of the user, to determine whether the measured body impedances are reasonable. When the impedance of the body is measured, the impedance of the body may be compared with a preset impedance range, to determine whether the measured impedance of the body is reasonable. When the body impedance falls within the preset impedance range, the measured body impedance is reasonable; otherwise, the measured body impedance is unreasonable. Further, when it is determined that the measured body impedance is unreasonable, the user may be prompted to perform measurement again. For example, the user may be prompted to perform the measurement again by using a voice. For example, after prompting the user, the wearable device 100 may redisplay a previous interface of a current display interface. For example, if the current display interface is (C) of FIG. 21, the interface shown in (B) of FIG. 21 is redisplayed; or if the current display interface is (E) of FIG. 21, the interface shown in (D) of FIG. 21 is redisplayed. In addition, on the interface shown in (E) of FIG. 21, the wearable device 100 may further determine fat content of the liver based on body impedances from two measurements, to obtain a risk level of the liver.

It may be understood that in a measurement process, the wearable device 100 may display different interfaces at different measurement stages, to prompt the user.

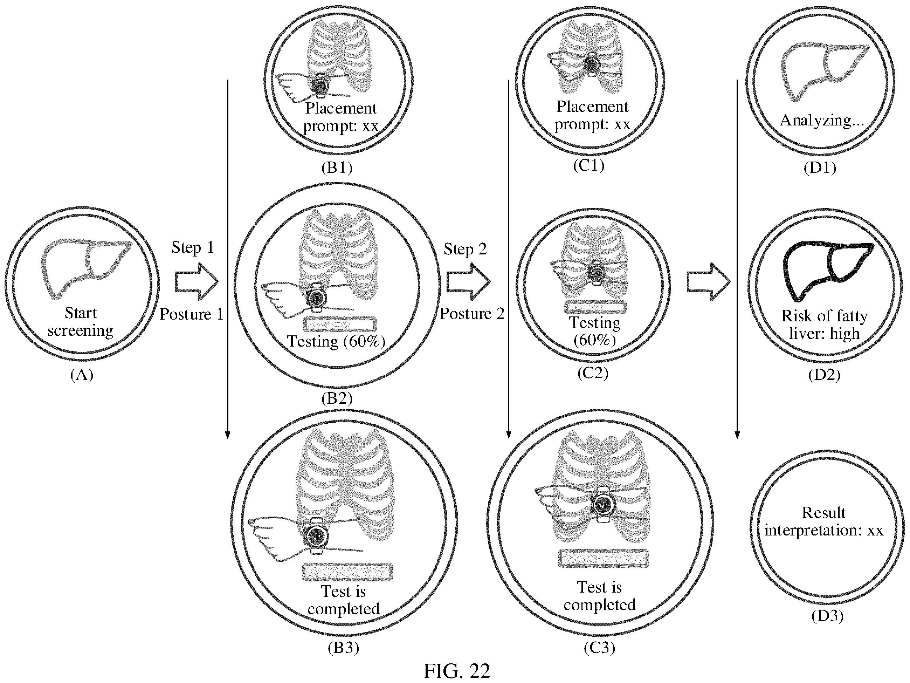

For example, after the user starts testing the risk level of the liver, the wearable device 100 may display an interface shown in (A) of FIG. 22, to prompt the user to start fatty liver screening.

Then the wearable device 100 may guide the user to perform testing by using a posture 1. In this process, the wearable device 100 may first display an interface shown in (B1) of FIG. 22, to visually prompt the user to place the wearable device 100 at a preset position or area on the user body. A placement prompt may be the same as the guide information b in (B) of FIG. 21. After the user places the wearable device 100 at the preset position or area on the user body, the wearable device 100 may display an interface shown in (B2) of FIG. 22, to prompt the user that body impedance measurement is being performed in the posture 1. After the wearable device measures a body impedance of the user in the posture 1, the wearable device 100 may display an interface shown in (B3) of FIG. 22, to prompt the user that measurement of the body impedance in the posture 1 has been completed. Then the wearable device 100 may guide the user to perform testing by using a posture 2.

In a process in which the wearable device 100 may guide the user to use the posture 2 for testing, the wearable device 100 may first display an interface shown in (C1) of FIG. 22, to visually prompt the user to place the wearable device 100 at a preset position or area on the user body. A placement prompt may be the same as the guide information c in (D) of FIG. 21. After the user places the wearable device 100 at the preset position or area on the user body, the wearable device 100 may display an interface shown in (C2) of FIG. 22, to prompt the user that body impedance measurement is being performed in the posture 2. After the wearable device measures a body impedance of the user in the posture 2, the wearable device 100 may display an interface shown in (C3) of FIG. 22, to prompt the user that measurement of the body impedance in the posture 2 has been completed.

After measuring body impedances of the user in different postures, the wearable device 100 may display an interface shown in (D1) of FIG. 22, to prompt the user that a liver risk level is being tested. After the risk level of the liver is tested, the wearable device 100 may display an interface shown in (D2) of FIG. 22, to display the risk level of the liver to the user. Then, the wearable device 100 may display an interface shown in (D3) of FIG. 22, to present a testing result interpretation to the user. The result interpretation may be the same as the result interpretation in (G) of FIG. 21. It should be noted that the display interfaces shown in FIG. 22 are merely examples for description, and do not constitute a limitation on this solution. The display interfaces may be selected according to a requirement, or may be redrawn according to a requirement. This is not limited herein.

In some embodiments, the wearable device 100 may alternatively send data measured by the wearable device 100 to another electronic device, so that the another electronic device obtains the risk level of the user's liver based on the data measured by the wearable device 100. For example, when the wearable device 100 is a smartwatch, the smartwatch may send impedance data of the liver measured by the smartwatch to a mobile phone. Then, the mobile phone may obtain a risk level of the liver based on the impedance data of the liver. In an example, the wearable device 100 may perform short-distance communication or long-distance communication with another electronic device, to implement information exchange between the wearable device 100 and the another electronic device. For example, a communication module may be disposed in the wearable device 100. The communication module may include at least one filter, a switch, a power amplifier, a low noise amplifier (low noise amplifier, LNA), and the like. The communication module may receive an electromagnetic wave through at least one antenna, perform processing such as filtering and amplification on the received electromagnetic wave, and transmit the electromagnetic wave to a modem for demodulation. The communication module may further amplify a signal modulated by the modem. The amplified signal is converted into an electromagnetic wave and radiated out via the antenna. In some examples, at least some function modules of the communication module may be disposed in the processor 13 of the wearable device 100. In some examples, at least some function modules of the communication module and at least some modules of the processor 13 of the wearable device 100 may be disposed in a same component. When the communication module includes a wireless communication module, the communication module may provide a wireless communication solution that is applied to the wearable device 100 and that includes a wireless local area network (wireless local area network, WLAN) (such as a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, and the like. The communication module may be one or more devices integrating at least one communication processing module. The communication module receives an electromagnetic wave via an antenna, performs frequency modulation and filtering processing on an electromagnetic wave signal, and sends the processed signal to the processor 13 of the wearable device 100. The communication module may further receive a to-be-sent signal from the processor 13 of the wearable device 100, and perform frequency modulation and amplification on the signal. The amplified signal is converted into an electromagnetic wave and radiated out via the antenna.

It may be understood that in embodiments of this application, in addition to the foregoing description of determining the risk level of the liver by using the liver impedance, an upper limb impedance of the user may be first measured, and then the risk level of the liver is determined based on the determined upper limb impedance. For details, refer to the following descriptions.

For example, when two groups of electrodes are disposed on the wearable device 100, when the upper limb impedance of the user is measured, the user may touch one group of electrodes of the wearable device 100 with a finger of the left hand, and touch another group of electrodes of the wearable device 100 with a finger of the right hand. Further, the two groups of electrodes on the wearable device 100 are electrically connected to each other. In this case, a current generated by the current generation unit 132 in the processor 13 of the wearable device 100 flows in a loop formed between the left hand and the right hand of the user. Then, the voltage measurement unit 132 in the processor 13 of the wearable device 100 may measure a voltage value between an electrode in contact with the left hand of the user and an electrode in contact with the right hand of the user. Finally, the control and processing unit 133 in the processor 13 of the wearable device 100 may obtain the upper limb impedance of the user based on the voltage value measured by the voltage measurement unit 132 and the current value generated by the current generation unit 131. In an example, the upper limb impedance may also be referred to as a first physiological parameter, the finger of the left hand of the user may also be referred to as a first part, and the finger of the right hand of the user may also be referred to as a second part.

After the upper limb impedance of the user is determined by using the wearable device 100, other physiological parameters of the user, for example, a body fat rate, a visceral fat amount in a torso, and a fat amount of a torso, may be obtained based on the upper limb impedance. In an example, physiological parameters such as a body fat rate, a visceral fat amount in a torso, and a fat amount of a torso may also be referred to as second physiological parameters.

For example, the body fat rate may be calculated by using the following formula. The formula (referred to as "Formula 5") is:

$$BFR = \alpha_1 R + \alpha_2 \quad \text{Formula 5}$$

*BFR* is the body fat rate, *R* is the upper limb impedance, and $\alpha_1$ and $\alpha_2$ are preset coefficients, and may be obtained through experiment.

In addition, when the upper limb impedance is measured by using a plurality of different frequencies of currents, the body fat rate may be calculated by using the following formula. The formula (referred to as "Formula 6") is:

$$BFR = \alpha_1 R_1 + \alpha_2 R_2 + \alpha_3 \quad \text{Formula 6}$$

*BFR* is the body fat rate; $R_1$ and $R_2$ are upper limb impedances measured under currents of different frequencies; and $\alpha_1$, $\alpha_2$ and $\alpha_3$ are preset coefficients, and may be obtained through experiment. It can be understood that, when a quantity of upper limb impedances measured under currents of different frequencies is greater than or equal to three,

"$\alpha_n R_n$" may be added to Formula 2. $R_n$ is an upper limb impedance measured under a current of the nth frequency, and $\alpha_n$ is a preset coefficient corresponding to $R_n$, and may be obtained through experiment.

The visceral fat amount in a torso may be calculated by using the following formula. The formula (referred to as "Formula 7") is:

$$X = \beta_1 R + \beta_2 \quad \text{Formula 7}$$

$X$ is the visceral fat amount in a torso; $R$ is the upper limb impedance; and $\beta_1$ and $\beta_2$ are preset coefficients, and may be obtained through experiment.

In addition, when the upper limb impedance is measured by using a plurality of different frequencies of currents, the visceral fat amount in a torso may be calculated by using the following formula. The formula (referred to as "Formula 8") is:

$$X = \beta_1 R_1 + \beta_2 R_2 + \beta_3 \quad \text{Formula 8}$$

$X$ is the visceral fat amount in a torso; $R_1$ and $R_2$ are upper limb impedances measured under currents of different frequencies; and $\beta_1$, $\beta_2$, and $\beta_3$ are preset coefficients, and may be obtained through experiment. It can be understood that, when a quantity of upper limb impedances measured under currents of different frequencies is greater than or equal to three, "$\beta_n R_n$" may be added to Formula 4. $R_n$ is an upper limb impedance measured under a current of the nth frequency, and $\beta_n$ is a preset coefficient corresponding to $R_n$, and may be obtained through experiment.

The fat amount of a torso may be calculated by using the following formula. The formula (referred to as "Formula 9") is:

$$P = \theta_1 R + \theta_2 \quad \text{Formula 9}$$

$P$ is the fat amount of a torso; $R$ is the upper limb impedance; and $\theta_1$ and $\theta_2$ are preset coefficients, and may be obtained through experiment.

In addition, when the upper limb impedance is measured by using a plurality of different frequencies of currents, the fat amount of a torso may be calculated by using the following formula. The formula (referred to as "Formula 10") is:

$$P = \theta_1 R_1 + \theta_2 R_2 + \theta_3 \quad \text{Formula 10}$$

$P$ is the fat amount of a torso; $R_1$ and $R_2$ are upper limb impedances measured under currents of different frequencies; and $\theta_1$, $\theta_2$ and $\theta_3$ are preset coefficients, and may be obtained through experiment. It can be understood that, when a quantity of upper limb impedances measured under currents of different frequencies is greater than or equal to three, "$\theta_n R_n$" may be added to Formula 6. $R_n$ is an upper limb impedance measured under the nth frequency, and $\theta_n$ is a preset coefficient corresponding to $R_n$, and may be obtained through experiment.

For example, when the physiological parameters such as the body fat rate, the visceral fat amount in a torso, and the fat amount of a torso are obtained based on the upper limb impedance, the physiological parameters may also be determined in combination with a body parameter such as a height of the user.

In this case, in combination with a height of the user, the body fat rate may be calculated by using the following formula. The formula (referred to as "Formula 11") is:

$$BFR = \alpha_1 R + \alpha_2 H + \alpha_3 \quad \text{Formula 11}$$

$BFR$ is the body fat rate, $R$ is the upper limb impedance, $H$ is the height; and $\alpha_1$, $\alpha_2$ and $\alpha_3$ are preset coefficients, and may be obtained through experiment.

In addition, when the upper limb impedance is measured by using a plurality of different frequencies of currents, the body fat rate may be calculated by using the following formula, in combination with a height of the user. The formula (referred to as "Formula 12") is:

$$BFR = \alpha_1 R_1 + \alpha_2 R_2 + \alpha_3 H + \alpha_4 \quad \text{Formula 12}$$

*BFR* is the body fat rate; $R_1$ and $R_2$ are upper limb impedances measured under currents of different frequencies; and $\alpha_1$, $\alpha_2$, $\alpha_3$ and $\alpha_4$ are preset coefficients, and may be obtained through experiment. It can be understood that, when a quantity of upper limb impedances measured under currents of different frequencies is greater than or equal to three, "$\alpha_n R_n$" may be added to Formula 2. $R_n$ is an upper limb impedance measured under a current of the n$^{th}$ frequency, and $\alpha_n$ is a preset coefficient corresponding to $R_n$, and may be obtained through experiment.

In combination with a height of the user, the visceral fat amount in a torso may be calculated by using the following formula. The formula (referred to as "Formula 13") is:

$$X = \beta_1 R + \beta_2 H + \beta_3 \quad \text{Formula 13}$$

*X* is the visceral fat amount in a torso; *R* is the upper limb impedance; and $\beta_1$, $\beta_2$, and $\beta_3$ are preset coefficients, and may be obtained through experiment.

In addition, when the upper limb impedance is measured by a plurality of different frequencies of currents, the visceral fat amount in a torso may be calculated by using the following formula in combination with a height of the user. The formula (referred to as "Formula 14") is:

$$X = \beta_1 R_1 + \beta_2 R_2 + \beta_3 H + \beta_4 \quad \text{Formula 14}$$

*X* is the visceral fat amount in a torso; $R_1$ and $R_2$ are upper limb impedances measured under currents of different frequencies; and $\beta_1$, $\beta_2$, $\beta_3$ and $\beta_4$ are preset coefficients, and may be obtained through experiment. It can be understood that, when a quantity of upper limb impedances measured under currents of different frequencies is greater than or equal to three, "$\beta_n R_n$" may be added to Formula 4. $R_n$ is an upper limb impedance measured under a current of the n$^{th}$ frequency, and $\beta_n$ is a preset coefficient corresponding to $R_n$, and may be obtained through experiment.

In combination with a height of the user, the fat amount of a torso may be calculated by using the following formula. The formula (referred to as "Formula 15") is:

$$P = \theta_1 R + \theta_2 H + \theta_3 \quad \text{Formula 15}$$

*P* is the fat amount of a torso, *R* is the upper limb impedance, and $\theta_1$, $\theta_2$ and $\theta_3$ are preset coefficients, and may be obtained through experiment.

In addition, when the upper limb impedance is measured by a plurality of different frequencies of currents, the fat amount of a torso may be calculated by using the following formula in combination with a height of the user. The formula (referred to as "Formula 16") is:

$$P = \theta_1 R_1 + \theta_2 R_2 + \theta_3 H + \theta_4 \quad \text{Formula 16}$$

*P* is the fat amount of a torso, $R_1$ and $R_2$ are upper limb impedances measured under currents of different frequencies; and $\theta_1$, $\theta_2$, $\theta_3$ and $\theta_4$ are preset coefficients, and may be obtained through experiment. It can be understood that, when a quantity of upper limb impedances measured under currents of different frequencies is greater than or equal to three, "$\theta_n R_n$" may be added to Formula 6. $R_n$ is an upper limb impedance measured under a current of the n$^{th}$ frequency, and $\theta_n$ is a preset coefficient corresponding to $R_n$, and may be obtained through experiment.

Further, after the physiological parameters such as the body fat rate, the visceral fat amount in a torso, and the fat amount of a torso are obtained, fat content of the user's liver may be determined in combination with a body mass index (body mass index, BMI) of the user. The user's BMI equals to $W/H^2$ , where W is a weight, and H is a height.

For example, the fat content of the user's liver may be calculated by using the following formula. The formula (referred to as "Formula 17") is:

$$M = \gamma_1 BMI + \gamma_2 BFR + \gamma_3 X + \gamma_4 P + \gamma_5 \quad \text{Formula 17}$$

*M* is the fat content of the liver; *BMI* is the body mass index; *BFR* is the body fat rate; X is the visceral fat amount in a torso; *P* is the fat amount of a torso; $\gamma_1$, $\gamma_2$, $\gamma_3$, $\gamma_4$ and $\gamma_5$ are preset coefficients, and may be obtained through experiment. It may be understood that the parameters (for example, *BMI, BFR, X,* and *P*) in Formula 13 may be selected according to a requirement, and this is not limited herein.

In addition, a waist circumference of the user may also be combined in calculation on the fat content of the user's liver. In this case, the fat content of the user's liver may be calculated by using the following formula. The formula (referred to as "Formula 18") is:

$$M = \gamma_1 BMI + \gamma_2 BFR + \gamma_3 X + \gamma_4 P + \gamma_5 L + \frac{\gamma_6 L}{H} + \gamma_7 \Delta T + \gamma_8 \quad \text{Formula 18}$$

*M* is the fat content of the liver; *BMI* is the body mass index; *BFR* is the body fat rate; *X* is the visceral fat amount in a torso; *P* is the fat amount of a torso; *L* is the waist circumference; and *H* is the height; *ΔT* is a timing variance between moments at which two waist circumferences are used, when a waist circumference used this time is different from a waist circumference used last time. When a waist circumference used this time is the same as a waist circumference used last time, or a timing variance between moments at which two waist circumferences are used exceeds a preset time threshold, or a waist circumference is used for the first time, *ΔT* may be 0. $\gamma_1$, $\gamma_2$, $\gamma_3$, $\gamma_4$, Ys , $\gamma_6$, $\gamma_7$, and $\gamma_8$ are preset coefficients, and may be obtained through experiment. It may be understood that the parameters (for example, *BMI, BFR, X, P, L, H* and *ΔT*) in Formula 14 may be selected according to a requirement, and this is not limited herein. In an example, the waist circumference and/or the height may also be referred to as a fourth physiological parameter.

After the fat content of the liver is obtained, the risk level of the user's liver may be determined based on a correspondence between the fat content of the liver and the risk level of the liver. For example, a preset correspondence between the fat content of the liver and the risk level of the liver may be shown in Table 1. When it is determined that fat content of the liver is "5", it can be learned from Table 1 that a risk level of the liver in this case is "suspicious risk". In an example, the fat content of the liver or the risk level of the liver may also be referred to as a third physiological parameter.

**Table 1**

| Liver fat content | Liver risk level |
|---|---|
| 0 to 4 | Normal |
| 4 to 7 | Suspected risk |
| 7 to 10 | Medium to high risk |

It may be understood that, in a process of testing the risk level of the liver by using the upper limb impedance, for an interface displayed on the wearable device 100, refer to the interface shown in FIG. 19. In this case, the guide information displayed in (B) of FIG. 19 is to guide the user to touch an electrode of the wearable device 100 with a finger. For a detailed display process, refer to the foregoing description. Details are not described herein again. It may be understood that, in embodiments of this application, in addition to displaying the risk level of the liver, the wearable device 100 may further display a physiological parameter such as a body fat rate of the user, so that the user learns of the physiological parameter such as the body fat rate of the user.

It may be understood that, the processor in embodiments of this application may be a central processing unit (central processing unit, CPU), the processor may further be another general-purpose processor, a digital signal processor (digital signal processor, DSP), an application-specific integrated circuit (application-specific integrated circuit, ASIC), a field programmable gate array (field programmable gate array, FPGA) or another programmable logic device, a transistor logic device, a hardware component, or any combination thereof. The general-purpose processor may be a microprocessor or any regular processor or the like.

The method steps in embodiments of this application may be implemented in a hardware manner, or may be implemented in a manner of executing software instructions by the processor. The software instructions may include corresponding software modules. The software modules may be stored in a random access memory (random access memory, RAM), a flash memory, a read-only memory (read-only memory, ROM), a programmable read-only memory (programmable ROM, PROM), an erasable programmable read-only memory (erasable PROM, EPROM), an electrically erasable programmable read-only memory (electrically EPROM, EEPROM), a register, a hard disk drive, a removable hard disk drive, a CD-ROM, or any other form of storage medium well-known in the art. For example, a storage medium is coupled to the processor, so that the processor can read information from the storage medium and write information into the storage medium. Certainly, the storage medium may be a component of the processor. The processor and the storage medium may be disposed in an ASIC.

All or some of the foregoing embodiments may be implemented by using software, hardware, firmware, or any combination thereof. When the software is used for implementing the embodiments, all or a part of the embodiments may be implemented in a form of a computer program product. The computer program product includes one or more computer instructions. When the computer program instructions are loaded and executed on a computer, the procedure or functions according to embodiments of this application are all or partially generated. The computer may be a general-purpose computer, a special-purpose computer, a computer network, or another programmable apparatus. The computer instructions may be stored in a computer-readable storage medium, or may be transmitted by using the computer-readable storage medium. The computer instructions may be transmitted from a website, a computer, a server, or a data center to another website, computer, server, or data center in a wired (for example, a coaxial cable, an optical fiber, or a digital subscriber line (DSL)) or wireless (for example, infrared, radio, or microwave) manner. The computer-readable storage medium may be any usable medium accessible by the computer, or a data storage device, for example, a server or a data center, integrating one or more usable media. The usable medium may be a magnetic medium (for example, a floppy disk, a hard disk drive, or a magnetic tape), an optical medium (for example, a DVD), a semiconductor medium (for example, a solid-state drive (solid-state disk, SSD)), or the like.

It may be understood that various numbers in embodiments of this application are merely used for differentiation for ease of description, but are not used for limiting the scope of embodiments of this application.

## Claims

1. A wearable device, comprising:

   a device body,
   a flexible fixing strip, connected to two ends of the device body, and
   at least two groups of electrodes, wherein each group of electrodes comprises at least one electrode, at least one group of the at least two groups of electrodes is disposed on the flexible fixing strip, and the at least two groups of electrodes are configured to measure a physiological parameter of a user based on an electrical signal generated by the wearable device.

2. The wearable device according to claim 1, wherein the at least two groups of electrodes comprise a first group of electrodes and a second group of electrodes, and the flexible fixing strip comprises a first flexible strip and a second flexible strip, wherein the first flexible strip is connected to one end of the device body, and the second flexible strip is connected to the other end of the device body; and
   the first group of electrodes is disposed on the first flexible strip, and the second group of electrodes is disposed on the second flexible strip.

3. The wearable device according to claim 2, wherein when both the first flexible strip and the second flexible strip are in an unfolded state, a distance between the first group of electrodes and the second group of electrodes is greater than a preset distance.

4. The wearable device according to claim 2 or 3, wherein the device body comprises a display screen, the first group of electrodes is located on a same side of the first flexible strip as the display screen, and the second group of electrodes is located on a side that is of the second flexible strip and that faces away from the display screen; or

   the first group of electrodes is located on a same side of the first flexible strip as the display screen, and the second group of electrodes is located on a same side of the second flexible strip as the display screen; or
   the first group of electrodes is located on a side that is of the first flexible strip and that faces away from the display screen, and the second group of electrodes is located on a side that is of the second flexible strip and that faces away from the display screen.

5. The wearable device according to claim 1, wherein the at least two groups of electrodes comprise a first group of electrodes and a second group of electrodes, the first group of electrodes is disposed on the device body, and the second group of electrodes is disposed on the flexible fixing strip.

6. The wearable device according to claim 5, wherein when the flexible fixing strip is in an unfolded state, a distance between the first group of electrodes and the second group of electrodes is greater than a preset distance.

7. The wearable device according to claim 5 or 6, wherein the device body comprises a display screen, the first group of electrodes is located on a side that is of the device body and that faces away from the display screen, and the second group of electrodes is located on a same side of the flexible fixing strip as the display screen; or

the first group of electrodes is located on a same side of the device body as the display screen, and the second group of electrodes is located on a same side of the flexible fixing strip as the display screen; or
the first group of electrodes is located on a side that is of the device body and that faces away from the display screen, and the second group of electrodes is located on a side that is of the flexible fixing strip and that faces away from the display screen; or
the first group of electrodes is located on a same side of the device body as the display screen, and the second group of electrodes is located on a side that is of the flexible fixing strip and that faces away from the display screen.

8. The wearable device according to any one of claims 1 to 7, wherein the flexible fixing strip is connected to the device body in an undetachable manner.

9. The wearable device according to claim 8, wherein the flexible fixing strip is provided with a first cable channel, the device body is provided with a second cable channel, the first cable channel and the second cable channel are communicated with each other and are both configured to accommodate a first cable, and the first cable is configured to connect a processor of the wearable device to the electrode on the flexible fixing strip.

10. The wearable device according to any one of claims 1 to 7, wherein the flexible fixing strip is detachably connected to the device body.

11. The wearable device according to claim 10, wherein the flexible fixing strip comprises a first locking piece, an elastic component, a pushing member, and a second locking piece, wherein the first locking piece, the elastic component, the pushing member, and the second locking piece are sequentially arranged in a width direction of the flexible fixing strip; and

the device body is provided with a first slot to which the first locking piece is fitted and a second slot to which the second locking piece is fitted, wherein
when external force toward the first locking piece is applied to the pushing member, the pushing member presses the elastic component, to drive the second locking piece to be disengaged from the second slot and/or move toward the first locking piece.

12. The wearable device according to claim 11, wherein the first locking piece is electrically connected to the at least one electrode on the flexible fixing strip, the first slot is electrically connected to a processor in the wearable device, and the first locking piece is electrically connected to the first slot; and/or
the second locking piece is electrically connected to the at least one electrode on the flexible fixing strip, the second slot is electrically connected to a processor in the wearable device, and the second locking piece is electrically connected to the second slot.

13. The wearable device according to any one of claims 1 to 12, wherein the at least two groups of electrodes are two groups, and each group of electrodes comprises one electrode, or each group of electrodes comprises two electrodes.

14. The wearable device according to any one of claims 1 to 13, wherein the physiological parameter comprises a liver impedance.

15. A physiological parameter measurement method, applied to a wearable device, wherein the wearable device comprises a device body, a flexible fixing strip, and at least two groups of electrodes, the flexible fixing strip is connected to two ends of the device body, the at least two groups of electrodes comprise a first group of electrodes and a second group of electrodes, the first group of electrodes is disposed on the flexible fixing strip, and the second group of electrodes is disposed on the flexible fixing strip or the device body; and
the method comprises:

detecting a first operation;
in response to the first operation, generating a first current when the first group of electrodes is electrically connected to the second group of electrodes, wherein the first current has a first current value, and the first current flows through the first group of electrodes and the second group of electrodes;

determining a first voltage value between the first group of electrodes and the second group of electrodes; and determining a first physiological parameter based on the first current value and the first voltage value.

**16.** The method according to claim 15, wherein the generating a first current;

determining a first voltage value between the first group of electrodes and the second group of electrodes; and determining a first physiological parameter based on the first current value and the first voltage value further comprises:

generating a second current of a first frequency, wherein the second current has a second current value; determining a second voltage value between the first group of electrodes and the second group of electrodes; generating a third current of a second frequency, wherein the third current has a third current value; determining a third voltage value between the first group of electrodes and the second group of electrodes; and

determining the first physiological parameter based on the second current value, the second voltage value, the third current value, and the third voltage value.

**17.** The method according to claim 15 or 16, wherein the device body comprises a display screen, and the first group of electrodes and the second group of electrodes are located on a same side of the flexible fixing strip and/or the device body as the display screen.

**18.** The method according to any one of claims 15 to 17, wherein the method further comprises:
in response to the first operation, displaying a first interface, wherein the first interface indicates a user to enable the first group of electrodes and the second group of electrodes to get in contact with a first position and a second position of a user body.

**19.** The method according to claim 18, wherein the method further comprises:

determining whether the first physiological parameter falls within a preset range; and
if the first physiological parameter is not within the preset range, prompting to perform re-measurement.

**20.** The method according to any one of claims 15 to 17, wherein the method further comprises:

in response to the first operation, displaying a second interface, wherein the second interface indicates a user to enable the first group of electrodes to get in contact with a first position of a user body; and
after the determining a first physiological parameter, the method further comprises:

displaying a third interface, wherein the third interface indicates the user to enable the first group of electrodes to get in contact with a second position of the user body;
generating a fourth current when the first group of electrodes is electrically connected to the second group of electrodes, wherein the fourth current has a fourth current value, and the fourth current flows through the first group of electrodes and the second group of electrodes;
determining a fourth voltage value between the first group of electrodes and the second group of electrodes;
determining a second physiological parameter based on the fourth current value and the fourth voltage value; and
determining a third physiological parameter based on the first physiological parameter and the second physiological parameter, wherein
the device body comprises the display screen, the second group of electrodes is located on a side that is of the device body and that faces away from the display screen, and the first group of electrodes is located on a same side of the flexible fixing strip as the display screen.

**21.** The method according to claim 20, wherein the method further comprises:

determining whether the third physiological parameter falls within a preset range; and
if the third physiological parameter is not within the preset range, prompting to perform re-measurement.

**22.** A wearable device, comprising:

at least one memory, configured to store a program; and

at least one processor, configured to execute the program stored in the memory, wherein when the program stored in the memory is executed, the processor is configured to perform the method according to any one of claims 15 to 21.

23. A computer storage medium, wherein the computer storage medium stores instructions, and when the instructions are run on a computer, the computer is enabled to perform the method according to any one of claims 15 to 21.

24. A computer program product comprising instructions, wherein when the instructions are run on a computer, the computer is enabled to perform the method according to any one of claims 15 to 21.

FIG. 1

FIG. 2

FIG. 3

FIG. 4

X direction

FIG. 5

FIG. 6

FIG. 7

FIG. 8

X direction

FIG. 9

FIG. 10

(A)

(B)

FIG. 11

Processor 13

Voltage measurement unit 132

Control and processing unit 133

Current generation unit 131

FIG. 12

FIG. 13

FIG. 14

FIG. 15

FIG. 16

(A)

(B)

FIG. 17

(A)

(B)

FIG. 18

Name: a
Height: 170
cm

a1

OK

(A)

Guide
information:
xx

(B)

Testing
(50%)

(C)

Result
interpretation:
xx

(E)

Test result:
xx

(D)

FIG. 19

Start
screening

(A)

Placement
prompt:
xx

(B)

Testing (60%)

(C)

Result
interpretation:
xx

(F)

Risk of fatty
liver: high

(E)

Analyzing...

(D)

FIG. 20

FIG. 21

FIG. 22

## INTERNATIONAL SEARCH REPORT

| International application No. |
| --- |
| **PCT/CN2022/101925** |

| **A.** | **CLASSIFICATION OF SUBJECT MATTER** |
| --- | --- |

A61B 5/0537(2021.01)i

According to International Patent Classification (IPC) or to both national classification and IPC

| **B.** | **FIELDS SEARCHED** |
| --- | --- |

Minimum documentation searched (classification system followed by classification symbols)

A61B5/-

Documentation searched other than minimum documentation to the extent that such documents are included in the fields searched

Electronic data base consulted during the international search (name of data base and, where practicable, search terms used)

CNTXT; CNABS; WPABS; VEN; 百度学术, BAIDU SCHOLAR; 读秀, DUXIU: 可穿戴, 手表, 手环, 胸戴, 脂肪, 内脏, 电流, 电压, 电极, 肝脏, 柔性带, 固定带, 显示屏, watch, bracelet, wristband, pole, intelligence, fat, liver, visceral.

| **C.** | **DOCUMENTS CONSIDERED TO BE RELEVANT** |
| --- | --- |

| Category* | Citation of document, with indication, where appropriate, of the relevant passages | Relevant to claim No. |
| --- | --- | --- |
| PX | CN 114041775 A (SUZHOU CHANGMAI TECHNOLOGY CO., LTD.) 15 February 2022 (2022-02-15) description, paragraphs 2-46, and figures 1-5 | 1-24 |
| X | CN 109875511 A (XU HONGZHE) 14 June 2019 (2019-06-14) description, paragraphs 2-44, and figures 1-5 | 1 |
| Y | CN 109875511 A (XU HONGZHE) 14 June 2019 (2019-06-14) description, paragraphs 2-44, and figures 1-5 | 2-24 |
| Y | CN 101152082 A (KAO CORP.) 02 April 2008 (2008-04-02) description, p. 15, paragraph 5 to p. 36, paragraph 3, and figures 1-22 | 2-4, 6-24 |
| Y | CN 111973174 A (HUAWEI TECHNOLOGIES CO., LTD.) 24 November 2020 (2020-11-24) description, paragraphs 1-69, and figures 1-8 | 2-24 |
| Y | CN 106255456 A (SHENZHEN GOODIX TECHNOLOGY CO., LTD.) 21 December 2016 (2016-12-21) description paragraphs 1-48, and figures 1-4 | 1-24 |
| Y | CN 102920454 A (BEIJING SHHC TECHNOLOGY CO., LTD.) 13 February 2013 (2013-02-13) description, paragraphs 2-70, and figures 1-8 | 1-24 |

☑ Further documents are listed in the continuation of Box C.    ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **12 September 2022** | **28 September 2022** |

| Name and mailing address of the ISA/CN | Authorized officer |
| --- | --- |
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2022/101925**

### C. DOCUMENTS CONSIDERED TO BE RELEVANT

| Category* | Citation of document, with indication, where appropriate, of the relevant passages | Relevant to claim No. |
|---|---|---|
| Y | CN 1957843 A (BENQ CORP.) 09 May 2007 (2007-05-09)<br>description, p. 1, paragraph 1 to p. 12, paragraph 6, and figures 1-10 | 1-24 |
| Y | CN 107811616 A (NINGBO INSTITUTE OF MATERIAL TECHNOLOGY AND ENGINEERING, CHINESE ACADEMY OF SCIENCES) 20 March 2018 (2018-03-20)<br>entire document | 1-24 |
| A | CN 200966617 Y (HE ZHIJIAN) 31 October 2007 (2007-10-31)<br>entire document | 1-24 |
| A | CN 107411730 A (ZOU HAIQING) 01 December 2017 (2017-12-01)<br>entire document | 1-24 |
| A | CN 1578499 A (BENQ CORP.) 09 February 2005 (2005-02-09)<br>entire document | 1-24 |
| A | CN 106249302 A (HUAWEI TECHNOLOGIES CO., LTD.) 21 December 2016 (2016-12-21)<br>entire document | 1-24 |
| A | CN 103815905 A (SICHUAN YUFENG SCIENCE & TECHNOLOGY DEVELOPMENT CO., LTD.) 28 May 2014 (2014-05-28)<br>entire document | 1-24 |
| A | CN 105455810 A (SOUTH CHINA UNIVERSITY OF TECHNOLOGY) 06 April 2016 (2016-04-06)<br>entire document | 1-24 |
| A | CN 108433724 A (SAMSUNG ELECTRONICS CO., LTD.) 24 August 2018 (2018-08-24)<br>entire document | 1-24 |
| A | KR 20180010719 A (INBODY CO., LTD.) 31 January 2018 (2018-01-31)<br>entire document | 1-24 |
| A | EP 3858230 A1 (CARDIOSCAN GMBH) 04 August 2021 (2021-08-04)<br>entire document | 1-24 |
| A | FR 2880113 A1 (SEB DEVELOPPEMENT) 30 June 2006 (2006-06-30)<br>entire document | 1-24 |

Form PCT/ISA/210 (second sheet) (January 2015)

## INTERNATIONAL SEARCH REPORT
### Information on patent family members

International application No.

**PCT/CN2022/101925**

| Patent document cited in search report | | | Publication date (day/month/year) | Patent family member(s) | | | Publication date (day/month/year) |
|---|---|---|---|---|---|---|---|
| CN | 114041775 | A | 15 February 2022 | None | | | |
| CN | 109875511 | A | 14 June 2019 | None | | | |
| CN | 101152082 | A | 02 April 2008 | CN | 1732846 | A | 15 February 2006 |
| | | | | CN | 1732845 | A | 15 February 2006 |
| CN | 111973174 | A | 24 November 2020 | None | | | |
| CN | 106255456 | A | 21 December 2016 | WO | 2017181330 | A1 | 26 October 2017 |
| CN | 102920454 | A | 13 February 2013 | None | | | |
| CN | 1957843 | A | 09 May 2007 | None | | | |
| CN | 107811616 | A | 20 March 2018 | None | | | |
| CN | 200966617 | Y | 31 October 2007 | None | | | |
| CN | 107411730 | A | 01 December 2017 | None | | | |
| CN | 1578499 | A | 09 February 2005 | None | | | |
| CN | 106249302 | A | 21 December 2016 | WO | 2018028180 | A1 | 15 February 2018 |
| CN | 103815905 | A | 28 May 2014 | None | | | |
| CN | 105455810 | A | 06 April 2016 | None | | | |
| CN | 108433724 | A | 24 August 2018 | US | 2018228432 | A1 | 16 August 2018 |
| KR | 20180010719 | A | 31 January 2018 | US | 2018020945 | A1 | 25 January 2018 |
| EP | 3858230 | A1 | 04 August 2021 | None | | | |
| FR | 2880113 | A1 | 30 June 2006 | CN | 101087557 | A | 12 December 2007 |
| | | | | BR | PI0519778 | A2 | 17 March 2009 |
| | | | | ES | 2532802 | T3 | 31 March 2015 |
| | | | | EP | 1827223 | A1 | 05 September 2007 |
| | | | | RU | 2007123586 | A | 27 December 2008 |
| | | | | PL | 1827223 | T3 | 29 May 2015 |
| | | | | PT | 1827223 | E | 17 March 2015 |
| | | | | WO | 2006070124 | A1 | 06 July 2006 |
| | | | | EP | 1827224 | A1 | 05 September 2007 |
| | | | | CN | 101080197 | A | 28 November 2007 |
| | | | | CN | 100479747 | C | 22 April 2009 |
| | | | | RU | 2394483 | C2 | 20 July 2010 |
| | | | | RU | 2397698 | C2 | 27 August 2010 |
| | | | | CN | 101080197 | B | 15 June 2011 |
| | | | | EP | 1827223 | B1 | 21 January 2015 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110769492